# EUROPEAN PATENT SPECIFICATION

(11) **EP 3 294 356 B1**
(45) Date of publication and mention of the grant of the patent: **07.07.2021**
(21) Application number: 16725382.2
(22) Date of filing: 09.05.2016
(51) Int. Cl.: A61L 27/24, A61L 27/38, A61L 27/60

(54) **COMPOSITIONS COMPRISING MESENCHYMAL STEM CELLS AND USES THEREOF**
ZUSAMMENSETZUNGEN MIT MESENCHYMALEN STAMMZELLEN UND VERWENDUNGEN DAVON
COMPOSITIONS COMPRENANT DES CELLULES SOUCHES MÉSENCHYMATEUSES ET LEURS UTILISATIONS

(30) Priority: 08.05.2015 US 201562158922 P; 31.07.2015 EP 15179418
(43) Date of publication of application: 21.03.2018
(73) Proprietor: Université Catholique de Louvain, 1348 Louvain-la-Neuve (BE)
(72) Inventor: DUFRANE, Denis, 1380 Lasne (BE)
(74) Representative: Savoye, Anne
(86) International application number: PCT/EP2016/060352
(87) International publication number: WO 2016/180789

(56) References cited:
- EP-A1- 2 374 485
- WO-A1-2014/150784
- US-A1- 2009 180 965

## Description

### FIELD OF INVENTION

The present invention relates to a biomedical product and its use for treating soft tissue injuries. In particular, the present invention relates to compositions comprising a mesenchymal stem cells population and a biocompatible matrix.

### BACKGROUND OF INVENTION

Every year, millions of people seek medical treatments for acute or overuse injuries of soft tissue, for example, injury to the skin (such as non-healing skin wounds) or to muscles (such as traumatic or surgical wounds). The process of wound healing follows a precise pattern including three phases: the inflammatory phase consisting of the release of cytokines for inflammatory response improvement; the repair or proliferation phase characterized by the activation and proliferation of progenitor cells under the influence of growth factors and cytokines and by angiogenesis; and the remodeling or maturation phase characterized by scar tissue and cross-links of collagen to other collagen and with protein molecules, increasing the tensile strength of the scar.

Several regenerative medicine approaches were proposed but remain limited by their clinical applications: local injection of growth factors remains associated with rapid depleted local concentration, inappropriate gradient, and loss of bioactivity (Borselli et al., Proc Natl Acad Sci U S A. 2010, 107:3287-3292); direct implantation of a decellularized extracellular matrix (containing native VEGF and bFGF) is associated with uncontrolled release of growth factors and lack of biocompatibility mainly due to the xenogenic origins (Keane et al., Biomaterials. 2012, 33:1771-1781); and the transplantation of differentiated muscles cells, for example, is limited by low cellular engraftment in the early phase after implantation due to hypoxic stress (Turner and Badylak, Cell Tissue Res. 2012, 347:759-774).

Stem cells were then proposed to improve the remodeling of ischemic muscular tissue by a high rate of proliferation and self-renewal, resistance to oxidative or hypoxic stress (Camassola et al., Methods Mol Biol Clifton NJ. 2012, 879:491-504), myogenesis, angiogenesis, and local cellular immune-modulation (Hong et al., Curr Opin Organ Transplant. 2010, 15:86-91).

Moreover, it has previously been demonstrated that stem cells seeded on a collagen matrix-containing tissue product enhances wound repair, in comparison of non-stem cells seeded on the same collagen matrix-containing tissue product (Lafosse et al., Plastic and Reconstructive Surgery 2015, 136(2):279-95).

Recent studies described three-dimensional scaffold seeded with mesenchymal stem cells, as disclosed in the European patent application EP 2374485 and in the US patent application US 2013/0121973. However, these patent applications are directed to differentiated mesenchymal stem cells, in particular into fibroblasts. Similarly, the international patent application WO 2014/150784 described a composition for treating soft tissue injury comprising a collagen matrix and cell suspension containing mesenchymal stem cells and non-mesenchymal stem cells.

However, these substitutes or compositions were not tested *in vivo,* in particular in humans.

Therefore, there still exists a need to improve the efficacy of current treatments for soft tissue injury. The Applicants surprisingly demonstrated that a substantially pure mesenchymal stem cells population may enhance repair and regeneration at the site of the injury in comparison to a mesenchymal stem cells population not substantially pure.

The scope of protection is defined by the claims.

Any references in the description to methods of treatments refer to the compositions of the present invention for use in a method for treatment of human or animal body.

Therefore, the present invention relates to a composition comprising a substantially pure mesenchymal stem cells population and a biocompatible matrix, as defined in claims 1-6), and said composition for use for treating soft tissue injuries in a subject in need thereof (claims 7-12).

### SUMMARY

This invention relates to a composition comprising a biocompatible matrix and a mesenchymal stem cells (MSC) population, wherein said mesenchymal stem cells population is substantially pure.

The mesenchymal stem cells population of the invention comprises less than 25% of fibroblasts.

The mesenchymal stem cells of the invention are undifferentiated.

The mesenchymal stem cells of the composition are derived from adipose tissue, more preferably from subcutaneous adipose tissue.

According to one embodiment, the biocompatible matrix of the invention is a cellular.

In one embodiment, the biocompatible matrix of the invention comprises collagen.

In one embodiment, the biocompatible matrix of the invention is human, porcine, bovine or equine.

According to one embodiment, mesenchymal stem cells of the invention originate from the subject to be treated.

The invention also relates to a composition comprising a biocompatible matrix and a mesenchymal stem cells population as described hereinabove for use for treating a soft tissue injury in a subject in need thereof.

According to one embodiment, the soft tissue to be treated in the invention is selected from the group comprising skin tissue, muscle tissue, dermal tissue, tendon tissue, ligament tissue, meniscus tissue and bladder tissue.

In one embodiment, the soft tissue injury is an acute or a chronic wound.

According to one embodiment, the soft tissue injury to be treated is selected from the group comprising tears or ruptures of soft tissue, skin wounds, skin burns, skin ulcers, surgical wounds, vascular diseases, muscle disease, hernias and radiation wounds. In a particular embodiment, the skin wound is a diabetic wound.

According to one embodiment, the composition for use of the invention is administered topically or by surgical implantation.

The invention also relates to a method for the preparation of a composition comprising a biocompatible matrix and a mesenchymal stem cells population according to claim 13.

### DEFINITIONS

In the present invention, the following terms have the following meanings:
- **"Mesenchymal stem cells"** or MSC, are multipotent stem cells which are capable of differentiating into more than one specific type of mesenchymal or connective tissue including osteogenic, chondrogenic, adipogenic, myelosupportive stroma, myogenic, or neurogenic lineages. Mesenchymal stem cells can be isolated from tissues including, without limitation, adipose tissue, bone-marrow, umbilical cord tissue, Wharton's jelly, amniotic fluid, placenta, peripheral blood, fallopian tube, corneal stroma, lung, muscle, skin, bone, dental tissue, pre-menstrual fluid, foreskin and fetal liver, and the like.
- **"Late passaged mesenchymal stem cell"** refers to a cell exhibiting a less immunogenic characteristic when compared to an earlier passaged cell. The immunogenicity of a mesenchymal stem cell corresponds to the number of passages. Preferably, the cell has been passaged up to at least the fourth passage, more preferably, the cell has been passaged up to at least the sixth passage, and most preferably, the cell has been passaged up to at least the eight passage.
- **"Normoxia"** refers to tissular or physiological oxygen levels. In one embodiment of the invention, cellular culture in normoxia, or in physiological oxygen levels, means at an oxygen level from about 3% to about 6%, preferably at an oxygen level of about 5%.
- **"Hypoxia"** refers to reduced oxygen levels. In one embodiment of the invention, cellular culture in hypoxia means at an oxygen level from about 0% to about 1%, preferably at an oxygen level of about 0.1%.
- **"Normoglycemia"** refers to normal levels of glucose. In one embodiment of the invention, cellular culture in normoglycemia means at a concentration of glucose of from about 0.5 g/l to about 1.5 g/l, preferably at a concentration of glucose of about 1 g/l.
- **"Hyperglycemia"** refers to elevated levels of glucose. In one embodiment of the invention, cellular culture in hyperglycemia means at a concentration of glucose from about 2 g/l to about 10 g/l, preferably from about 3 g/l to about 6 g/l, more preferably at a concentration of glucose of about 4.5 g/l.
- **"Biocompatible"** refers to the quality of not having toxic or injurious effects on the body, in particular on a soft tissue. In one embodiment, the biocompatibility of a material refers to the ability of said material to perform its desired function without eliciting any undesirable local or systemic effects in the subject, but generating the most appropriate beneficial cellular or tissue response.
- **"Biocompatible matrix",** also referred as matrix or scaffold, refers to a three-dimensional scaffold formed by biocompatible material.
- **"Acellular",** or "decellularized", with reference to a biocompatible matrix, refers to a matrix from which substantially all endogenous cells have been removed, such as, for example, at least about 60% of endogenous cells (wherein the percentages are relative to the number of endogenous cells), preferably about 65, 70, 75, 80, 85, 90, 95, 96, 97, 98, 99% of endogenous cells or more. The decellularization of the matrix may be evaluated by counting viable cells, for example by DAPI staining.
- **"Soft tissue"** refers to the tissues that connect, support, or surround other structures and organs of the body, not being bone. In one embodiment, soft tissue includes, without limitation, tendons, ligaments, fascia, skin, fibrous tissues, fat, and synovial membranes (which are connective tissue), muscles, nerves and blood vessels (which are not connective tissue). In another embodiment, soft tissues are body tissues except bone, teeth, nails, hair, and cartilage.
- **"Subject"** refers to a mammal, preferably a human. In one embodiment, a subject may be a "patient", i.e. a warm-blooded animal, more preferably a human, who/which is awaiting the receipt of, or is receiving medical care or was/is/will be the object of a medical procedure, or is monitored for the development or the healing of a soft tissue injury. In one embodiment, the subject is an adult (for example a subject above the age of 18). In another embodiment, the subject is a child (for example a subject below the age of 18). In one embodiment, the subject is a male. In another embodiment, the subject is a female.
- **"Treating"** or "treatment" or "alleviation" refers to therapeutic action taken and action refrained from being taken for the purpose of improving the condition of the patient, wherein the object is to slow down (lessen) or to reverse the progress, or to alleviate one or more symptoms of the soft tissue injury such as, for example, unclosed wound, fibrosis development, lack of vascularization and inflammation. A subject or mammal is successfully "treated" for a soft tissue injury if, after receiving a composition according to the present invention, the patient shows observable and/or measurable reduction in or absence of one or more of the following: relief to some extent, of one or more of the symptoms associated with the soft tissue injury; reduced morbidity; reduced mortality, or improvement in quality of life. The above parameters for assessing successful treatment and improvement in the injury are readily measurable by routine procedures familiar to a physician.
- **"Passaging",** also known as subculture or splitting cells, refers to transferring a small number of cells into a new vessel when cells are at confluence or almost, to prolong the life and/or expand the number of cells in the culture. In one embodiment, the passage 0 (P0) is the point at which cells were initially placed in culture.
- **"About"** and **"Approximately"** as used herein when referring to a measurable value such as an amount of a compound, dose, time, temperature, and the like, is meant to encompass variations of 20%, 10%, 5%, 1%, 0.5%, or even 0.1% of the specified amount.
- **"And/Or"** refers to and encompasses any and all possible combinations of one or more of the associated listed items, as well as the lack of combinations when interpreted in the alternative ("or").
- **"Derivative"** as used herein refers to any fraction of a substance, derivative, subfamily, etc., or mixtures thereof, alone or in combination with other derivatives or other ingredients.
- **"Isolated"** as used herein signifies that the cells are placed into conditions other than their natural environment.

### DETAILED DESCRIPTION

Previously described mesenchymal stem cells (MSC) populations comprise other types of cells than mesenchymal stem cells, in particular they may comprise fibroblasts, or are in part differentiated into fibroblasts. The Applicant herein demonstrates that mesenchymal stem cells have the capacity to better survive and proliferate in hypoxia and/or hyperglycemia than fibroblasts. Moreover, mesenchymal stem cells have the capacity to secrete more VEGF than fibroblasts, in particular mesenchymal stem cells secrete more VEGF in hypoxia and hyperglycemia conditions (i.e. diabetic wounds conditions) than in normoxia and normoglycemia (see Example 2).

The Applicants also demonstrates that mesenchymal stem cells seeded on a biocompatible matrix may restore dermal tissue (see Example 3), and may be used for the treatment of injured muscle (see Example 4).

Moreover, a MSC population seeded on a biocompatible matrix demonstrated the capacity to survive under hypoxia and/or hyperglycemia, to improve the release of pro-angiogenic factors by an oxygen-sensitive mechanism and to reduce fibrotic scar. These properties could promote soft tissue reconstruction, such as, for example, in diabetic conditions.

This invention thus relates to a composition comprising a biocompatible matrix and a mesenchymal stem cells (MSC) population.

The composition of the invention may be used in tissue engineering and regeneration in animals. The exact composition of the invention may vary according to the use desired. Modifications and other embodiments of the invention will become apparent to one skilled in the art to which this invention pertains having the benefit of the teachings presented in the foregoing descriptions and associated drawings. It is to be understood that the invention is not limited to the specific embodiments disclosed and that modifications and other embodiments are intended to be included within the scope of the appended claims.

In one embodiment, the composition of the invention is a biomedical product.

According to the disclosure, the MSC population may comprise other types of cells than MSC, such as, for example, fibroblasts, macrophages, endothelial cells, dendritic cells, osteoblasts, hematopoietic stem cells, cord blood stem cells, lymphocytes, or natural killer cells.

Cells may be obtained from a donor (either living or cadaveric) or derived from an established cell strain or cell line. For example, cells may be harvested from a donor using standard biopsy techniques known in the art.

In one embodiment, MSC are obtained from a human donor.

In one embodiment, MSC are obtained from a human donor, provided that they are not embryonic stem cells.

In one embodiment of the invention, the MSC population is an isolated MSC population.

The MSC population is substantially pure. As used herein, the term "substantially pure MSC population" means that the MSC population comprises less than 25% of other types of living cells, in particular of fibroblasts. In one embodiment, the substantially pure MSC population of the invention comprises at least about 75%, 80, 85, 90, 91, 92, 93, 94, 95, 96, 97, 98, 99, or 100% of MSC, wherein percentages are relative to the total number of living cells.

In another embodiment, the substantially pure MSC population of the invention comprises less than about 25, 24, 23, 22, 21, 20, 19, 18, 17, 16, 15, 14, 13, 12, 11, 10, 9, 8, 7, 6, 5, 4, 3, 2 or 1% of fibroblasts, wherein percentages are relative to the total number of living cells.

According to one embodiment, the substantially pure MSC population of the invention secretes at most 100 pg/ml of SDF-1α, preferably at most 50, 40, 30, 25, 20, 19, 18, 17, 16, 15, 14, 13, 12, 11, 10, 9, 8, 7, 6, 5, 4, 3, 2 or 1 pg/ml.

According to one embodiment, the substantially pure MSC population of the invention secretes at most 50 pg/ml of SDF-1α, preferably at most 40, 30, 25, 20, 19, 18, 17, 16, 15, 14, 13, 12, 11, 10, 9, 8, 7, 6, 5, 4, 3, 2 or 1 pg/ml, when the MSC population is cultured at least 24 hours at about 21% O₂.

According to another embodiment, the substantially pure MSC population of the invention secretes at most 50 pg/ml of SDF-1α, preferably at most 40, 30, 25, 20, 19, 18, 17, 16, 15, 14, 13, 12, 11, 10, 9, 8, 7, 6, 5, 4, 3, 2 or 1 pg/ml, when the MSC population is cultured at least 24 hours in physiological oxygen levels, preferably at about 5% O₂.

According to another embodiment, the substantially pure MSC population of the invention secretes at most 50 pg/ml of SDF-1α, preferably at most 40, 30, 25, 20, 19, 18, 17, 16, 15, 14, 13, 12, 11, 10, 9, 8, 7, 6, 5, 4, 3, 2 or 1 pg/ml, when the MSC population is cultured at least 24 hours in hypoxia, preferably at about 0.1% O₂.

According to another embodiment, the substantially pure MSC population of the invention secretes at most 100 pg/ml of SDF-1α, preferably at most 50, 40, 30, 25, 20, 19, 18, 17, 16, 15, 14, 13, 12, 11, 10, 9, 8, 7, 6, 5, 4, 3, 2 or 1 pg/ml of SDF-1α, when the MSC population is cultured at least 24 hours in normoglycemia, preferably at a concentration of glucose of about 1 g/l of glucose.

According to another embodiment, the substantially pure MSC population of the invention secretes at most 50 pg/ml of SDF-1α, preferably at most 40, 30, 25, 20, 19, 18, 17, 16, 15, 14, 13, 12, 11, 10, 9, 8, 7, 6, 5, 4, 3, 2 or 1 pg/ml of SDF-1α, when the MSC population is cultured at least 24 hours in hyperglycemia, preferably at a concentration of glucose of about 4.5 g/l.

In one embodiment, the substantially pure MSC population of the invention secretes at most of 100 pg/ml, preferably at most of 50, 40, 30, 25, 20, 19, 18, 17, 16, 15, 14, 13, 12, 11, 10, 9, 8, 7, 6, 5, 4, 3, 2 or 1 pg/ml of SDF-1α, when the MSC population is cultured at least 24 hours at about 21% O₂ and at low concentration of glucose, preferably at about 1 g/l of glucose.

In another embodiment, the substantially pure MSC population of the invention secretes at most of 50 pg/ml, preferably at most of 40, 30, 25, 20, 19, 18, 17, 16, 15, 14, 13, 12, 11, 10, 9, 8, 7, 6, 5, 4, 3, 2 or 1 pg/ml of SDF-1α, when the MSC population is cultured at least 24 hours at about 21% O₂ and at high concentration of glucose, preferably at about 4.5 g/l of glucose.

In another embodiment, the substantially pure MSC population of the invention secretes at most of 100 pg/ml, preferably at most of 50, 40, 30, 25, 20, 19, 18, 17, 16, 15, 14, 13, 12, 11, 10, 9, 8, 7, 6, 5, 4, 3, 2 or 1 pg/ml of SDF-1α, when the MSC population is cultured at least 24 hours in physiological oxygen levels, preferably at about 5% O₂, and at low concentration of glucose, preferably at about 1 g/l of glucose.

In another embodiment, the substantially pure MSC population of the invention secretes at most of 50 pg/ml, preferably at most of 40, 30, 25, 20, 19, 18, 17, 16, 15, 14, 13, 12, 11, 10, 9, 8, 7, 6, 5, 4, 3, 2 or 1 pg/ml of SDF-1α, when the MSC population is cultured at least 24 hours in physiological oxygen levels, preferably at about 5% O₂, and at high concentration of glucose, preferably at about 4.5 g/l of glucose.

In another embodiment, the substantially pure MSC population of the invention secretes at most of 100 pg/ml, preferably at most of 50, 40, 30, 25, 20, 19, 18, 17, 16, 15, 14, 13, 12, 11, 10, 9, 8, 7, 6, 5, 4, 3, 2 or 1 pg/ml of SDF-1α, when the MSC population is cultured at least 24 hours in hypoxia, preferably at about 0.1% O₂, and at low concentration of glucose, preferably at about 1 g/l of glucose.

In another embodiment, the substantially pure MSC population of the invention secretes at most of 50 pg/ml, preferably at most of 40, 30, 25, 20, 19, 18, 17, 16, 15, 14, 13, 12, 11, 10, 9, 8, 7, 6, 5, 4, 3, 2 or 1 pg/ml of SDF-1α, when the MSC population is cultured at least 24 hours in hypoxia, preferably at about 0.1% O₂, and at high concentration of glucose, preferably at about 4.5 g/l of glucose.

In one embodiment, the substantially pure MSC population of the invention secretes at least 200 pg/ml of VEGF, preferably at least about 250, 260, 270, 280, 281, 282, 283, 284, 285, 286, 287, 288, 289 or 290 pg/ml of VEGF, when the MSC population is cultured at least 24 hours in hypoxia, preferably at about 0.1% O₂, and hyperglycemia, preferably at a concentration of glucose of about 4.5 g/l.

In another embodiment, the substantially pure MSC population of the invention secretes at least about 90 pg/ml of VEGF, preferably at least about 95, 100, 105, 110, 111, 112, 113, 114, 115, 116, 117, 188, 119 or 120 pg/ml of VEGF, when the MSC population is cultured at least 24 hours in physiological oxygen levels, preferably at about 5% O₂, and hyperglycemia, preferably at a concentration of glucose of about 4.5 g/l.

In another embodiment, the substantially pure MSC population of the invention secretes at least about 160 pg/ml of VEGF, preferably at least about 161, 162, 163, 164, 165, 166, 167, 168, 169 or 170 pg/ml of VEGF, when the MSC population is cultured at least 24 hours in physiological oxygen levels, preferably at about 5% O₂, and normoglycemia, preferably at a concentration of glucose of about 1 g/l.

In one embodiment, the substantially pure MSC population of the invention secretes at most 50 pg/ml of SDF-1α, preferably at most 40, 30, 25, 20, 19, 18, 17, 16, 15, 14, 13, 12, 11, 10, 9, 8, 7, 6, 5, 4, 3, 2 or 1 pg/ml of SDF-1α; and at least about 200 pg/ml of VEGF, preferably at least about 250, 260, 270, 280, 281, 282, 283, 284, 285, 286, 287, 288, 289 or 290 pg/ml of VEGF, when the MSC population is cultured at least 24 hours in hypoxia, preferably at about 0.1% O₂, and hyperglycemia, preferably at a concentration of glucose of about 4.5 g/l.

In another embodiment, the substantially pure MSC population of the invention secretes at most 50 pg/ml of SDF-1α, preferably at most 40, 30, 25, 20, 19, 18, 17, 16, 15, 14, 13, 12, 11, 10, 9, 8, 7, 6, 5, 4, 3, 2 or 1 pg/ml of SDF-1α; and at least about 90 pg/ml of VEGF, preferably at least about 95, 100, 105, 110, 111, 112, 113, 114, 115, 116, 117, 188, 119 or 120 pg/ml of VEGF, when the MSC population is cultured at least 24 hours in physiological oxygen levels, preferably at about 5% O₂, and hyperglycemia, preferably at a concentration of glucose of about 4.5 g/l.

In another embodiment, the substantially pure MSC population of the invention secretes at most 100 pg/ml of SDF-1α, preferably at most 50, 40, 30, 25, 20, 19, 18, 17, 16, 15, 14, 13, 12, 11, 10, 9, 8, 7, 6, 5, 4, 3, 2 or 1 pg/ml of SDF-1α; and at least about 160 pg/ml of VEGF, preferably at least about 161, 162, 163, 164, 165, 166, 167, 168, 169 or 170 pg/ml of VEGF, when the MSC population is cultured at least 24 hours in physiological oxygen levels, preferably at about 5% O₂, and in normoglycemia, preferably at a concentration of glucose of about 1 g/l of glucose.

According to one embodiment, the MSC population is cultured in normoxia, preferably at about 21% O₂, and in normoglycemia, preferably at about 1 g/l of glucose, up to at least 1, 2, 3 or 4 passages before measuring the SDF-1α and/or VEGF expression level. According to one embodiment, the MSC population is cultured up to confluence of the MSC before measuring the SDF-1α and/or VEGF expression level. In another embodiment, the MSC population is cultured up to at least about 80, 85, 90, 95, 99, or 100% confluence of MSC before measuring the SDF-1α and/or VEGF expression level.

The mesenchymal stem cells are undifferentiated, or non-differentiated, i.e. MSC are not differentiated into a cellular type, in particular into fibroblasts.

MSC may be isolated from tissues selected from the group comprising adipose tissue, bone marrow, umbilical cord blood, Wharton's jelly (such as, for example, Wharton's jelly found within the umbilical cord), amniotic fluid, placenta, peripheral blood, fallopian tube, corneal stroma, lung, muscle, skin, bone, dental tissue and fetal liver, or the like. The MSC of the invention are isolated from adipose tissue. The MSC of the invention are adipose stem cells (referred to as ASC, or as AMSCs).

In one embodiment, MSC are isolated from any warm-blooded animal tissues, preferably from human, porcine, bovine or equine tissues, more preferably from human tissues. In a particular embodiment, MSC are human ASC.

In one embodiment, the cells are cells in culture, preferably are cell lines and/or are derived from primary cells, i.e. cells isolated straight from the tissue. In one embodiment, the cells are recovered from a sample from an individual, obtained for example by biopsy. Preferably, the step of recovering the sample from an individual is not part of the method of the present invention.

Isolation of mesenchymal stem cells may be accomplished by any acceptable method known to one of ordinary skill in the art. Examples of methods for isolating MSC include, but are not limited to, digestion by collagenase, trypsinization, or explant culture.

In a particular embodiment, mesenchymal stem cells are isolated from adipose tissue by digestion of the tissue, for example by collagenase.

In one embodiment, the MSCs of the invention may be stably or transiently transfected or transduced with a nucleic acid of interest using suitable a plasmid, viral or alternative vector strategy. Nucleic acids of interest include, but are not limited to, those encoding gene products which enhance the production of extracellular matrix components found in the tissue type to be repaired.

According to one embodiment of the invention, after isolation, the MSC population is cultured in any culture medium designed to support the growth of the cells known to one of ordinary skill in the art. As used herein, such culture medium is called "proliferation medium" or "growth medium". Examples of growth medium include, without limitation, MEM, DMEM, CMRL, IMDM, RPMI 1640, FGM or FGM-2, 199/109 medium, HamF10/HamF12 or McCoy's 5A, preferably DMEM or RPMI.

In one embodiment, the culture medium may further comprise any supplementary factors. Examples of supplementary factors include, but are not limited to, FBS; platelet lysate; glycine; amino acids, such as glutamine, asparagine, glutamic acid, aspartic acid, serine, proline or alanine, preferably the L-configuration of amino acids; and antibiotics, such as streptomycin or penicillin.

According to one embodiment, the MSC population is cultured in standard culture conditions. As used herein, "standard culture conditions" means at a temperature of 37°C and in 5% CO₂.

The MSC population of the invention is not differentiated into a specific tissue. Accordingly, in one embodiment, the MSC population of the invention is not cultured in conditions to induce differentiation, such as, for example, in a differentiation medium. In a particular embodiment, the MSC population of the invention is not cultured in osteogenic differentiation medium, muscle differentiation medium or dermal differentiation medium.

In one embodiment, a substantially pure MSC population is obtained by culturing the MSC population up to at least 3 passages, preferably in normoxia and normoglycemia.

In one embodiment, the MSC population may be frozen, preferably at least after the 3th passage. As an example, the cells population may be frozen and stored in liquid nitrogen or at any temperature, preferably from about -0°C to -196°C, so long as the cells are able to be used as stem cells after thawing therefrom. In one embodiment, the MSC population may be thawed and expanded further to obtain fresh cells.

In one embodiment, the biocompatible matrix is formed by bioresorbable biological material. As used herein, the term "bioresorbable" means that the biological material can be assimilated back, dissolved or absorbed in the body and does not require mechanical or manual removal.

In one embodiment, the biocompatible matrix of the invention is formed by a material that provides a structural support for the growth and propagation of cells. In one embodiment, biocompatible matrix comprises a natural or synthetic material, or a chemical-derivative thereof, selected from the group comprising agar/agarose, alginates chitosan, chondroitin sulfate, collagen, elastin or elastin-like peptides (ELP), fibrinogen, fibrin, fibronectin, gelatin, heparan sulfate proteoglycans, hyaluronic acid, polyanhydrides, polylactic acid (PLA), poly(lactic-co-glycolic acid) (PLGA), polyethylene oxide/ polyethylene glycol (PEO/PEG), poly(vinyl alcohol) (PVA), fumarate-based polymers such as, for example poly(propylene fumarate) (PPF) or poly(propylene fumarate-co-ethylene glycol) (P(PF-co-EG)), oligo(poly(ethylene glycol) fumarate) (OPF), poly (n-isopropylacrylamide) (PNIPPAAm), poly(aldehyde guluronate) (PAG), polyanhydrides, poly(n-vinyl pyrrolidone) (PNVP), self-assembling oligopeptide gels, and/or any other suitable hydrogel material as a cell carrier to complement tissue growth. In a particular embodiment, the biocompatible matrix of the invention comprises collagen, such as, for example, type I collagen.

According to one embodiment, the biocompatible matrix of the invention is derived from any collagenous tissues. Examples of collagenous tissues include, but are not limited to, skin, dermis, tendon, ligament, muscle, amnion, meniscus, small intestine submucosa, cardiac valve, vessel and bladder. In a particular embodiment, the biocompatible matrix of the invention is derived from tendon, preferably from fascia lata.

In one embodiment, the biocompatible matrix is recovered from an individual. In an embodiment, the biocompatible matrix is recovered from a human. In another embodiment, the biocompatible matrix is recovered from a non-human animal. Such biocompatible matrix are commercially available. Examples of commercially available biocompatible matrix include, but are not limited to, Integra® (Integra LifeSciences Corporation), Matriderm® (Skin & Health Care), Alloderm® (Life Cell) and Puramatrix® (3-D Matrix Medical Technology). Preferably, the step of recovering the biocompatible matrix from an individual is not part of the method of the present invention.

In a particular embodiment of the invention, the biocompatible matrix is derived from human fascia lata tendon.

In one embodiment, the biocompatible matrix of the invention has low or no immunogenicity. In one embodiment, the biocompatible matrix is processed, by any biological, chemical and/or physical means to reduce its immunogenicity. After a process to reduce immunogenicity, the biocompatible matrix of the invention is less immunogenic in comparison to unprocessed biocompatible matrix of the same type.

In one embodiment, to reduce immunogenicity the biocompatible matrix is processed to remove cellular membranes, nucleic acids, lipids and cytoplasmic components. After the process to reduce immunogenicity, the biocompatible matrix comprises intact the components typically associated with the matrix, such as, for example, collagen, elastins, glycosaminoglycans, and proteoglycans.

In one embodiment, the biocompatible matrix is processed to reduce immunogenicity. In one embodiment, the biocompatible matrix of the invention is at least about 50%, 60%, 70%, 80% or 90% less immunogenic than unprocessed biocompatible matrix of the same type. The parameters for assessing the immunogenicity of a matrix are readily measurable by routine procedures familiar to a physician.

Examples of processes to reduce immunogenicity include, but are not limited to, decellularization of the biocompatible matrix and cellular disruption of the biocompatible matrix.

In one embodiment, the biocompatible matrix of the invention has been processed with a cellular disruption method. Examples of cellular disruption methods include, but are not limited to, cryopreservation, freeze/thaw cycling, and exposure to radiation.

In another embodiment, the biocompatible matrix of the invention has been processed with to a decellularization method. As used herein, "a decellularization method" means a method to remove endogenous cells from the biocompatible matrix by use of physical, chemical, or biochemical means. Examples of means include, but are not limited to, enzymes such as proteases and/or nucleases; chemicals such as acids, bases, ionic detergents, non-ionic detergents, surfactants, and/or zwitterionic detergents; defatting agents such as acetone; and/or freeze-drying.

In one embodiment, the biocompatible matrix of the invention is acellular. In one embodiment, the biocompatible matrix comprises at most about 40, 35, 30, 25, 20, 19, 18, 17, 16, 15, 14, 13, 12, 11, 10, 9, 8, 7, 6, 5, 4, 3, 2, 1% or less of endogenous cells, in comparison of a biocompatible matrix of the same type which has not been subjected to removal of endogenous cells.

Decellularization may be quantified according to any method known from the person skilled in the art. Examples of methods of quantifying decellularization include, but are not limited to, staining with 40,6-diamidino-phenylindole (DAPI), or measuring DNA concentration in the processed biocompatible matrix, in comparison with the biocompatible matrix before processing or with a biocompatible matrix of the same type which has not been processed.

In a preferred embodiment, the biocompatible matrix is an acellular collagen matrix, preferably a human acellular collagen matrix (HACM).

In one embodiment, the biocompatible matrix is prepared to obtain a matrix comprising no detectable chemical residues, such as, for example, acetone or H₂O₂, and less than about 10% of residual moisture, preferably less than 8%. Such preparation of the biocompatible matrix may be accomplished by any acceptable method known to one of ordinary skill in the art. Thus, in one embodiment of the invention, the biocompatible matrix comprises no chemical residues, such as, for example, acetone or H₂O₂, and less than about 10% of residual moisture, preferably less than 8%.

In another embodiment, the biocompatible matrix is sterilized to avoid infectious disease transmission, such as, for example, HIV, hepatitis C and B viruses (HCV, HBV) and bacterial infections. Examples of sterilization methods include, but are not limited to, chemicals, heat, UV and ionizing radiation, such as gamma irradiation.

In one embodiment, the biocompatible matrix and the MSC population of the invention are from same species. In another embodiment, the biocompatible matrix and the MSC population of the invention are from different species. In another embodiment, the biocompatible matrix and the MSC population of the invention are from the same donor subject, who may, or may not be the recipient subject. In another embodiment, the biocompatible matrix and the MSC population of the invention are from different subjects, either one or neither, may be the recipient subject.

The mesenchymal stem cells population is incubated with the biocompatible matrix. As used herein, the term "incubated" means that the mesenchymal stem cells population contacts the biocompatible matrix.

In one embodiment, the mesenchymal stem cells population may be seeded on the biocompatible matrix. In another embodiment, the mesenchymal stem cells population may be seeded into the biocompatible matrix. In another embodiment, the mesenchymal stem cells population may be seeded on and into the biocompatible matrix.

In one embodiment, the cells in the composition may be seeded in any arrangement. For example, the cells may be distributed homogeneously throughout the biocompatible matrix or distributed in defined zones, regions or layers within the biocompatible matrix.

Seeding of the cells may be preferably performed under suitable conditions of temperature, pH, ionic strength and sheer to maintain cell viability.

In one embodiment, the MSC population is cultured up to at least passage 2, 3, 4 or 5 before being incubated with the biocompatible matrix. As used herein, the term "cultured up to at least passage 4" means that the cell population is detached and transferred into a new vessel up to at least 4 times. In a particular embodiment, the MSC population is cultured up to passage 4 before being incubated with the biocompatible matrix.

In one embodiment, the mesenchymal stem cells incubated with the biocompatible matrix are late passaged mesenchymal stem cells.

In one embodiment, the MSC population is passaged when the MSC population reaches about 70, 75, 80, 85, 90, 95, 99, or 100% confluence.

In another embodiment, the MSC population is cultured for at least 24 hours, preferably for at least 36, 48, 60 or 72 hours before being incubated with the biocompatible matrix. In another embodiment, the MSC population is cultured for at least 1 day, 2, 3, 4, 5, 6, 7, 8, 9, 10, 15, 20, 25, 30, 35, 40 days before being incubated with the biocompatible matrix.

In some embodiments, the biocompatible matrix is incubated with the MSC population in 12-well or 24-well plates, or in cell culture flasks of 25 cm², 75 cm² or 150 cm².

In one embodiment, the MSC population is counted and initially incubated with the biocompatible matrix at a density of at least 0.5x10⁵, 1x10⁵, 2x10⁵ or 3x10⁵ cells per well. In another embodiment, the MSC population is initially incubated with the biocompatible matrix at a density from about 0.5x10⁵ to about 5x10⁵ cells/well (per well), preferably from about 1x10⁵ to about 4x10⁵ cells/well, more preferably from about 2x10⁵ to about 3x10⁵ cells/well. In a preferred embodiment, the MSC population is initially incubated with the biocompatible matrix at a density of about 2x10⁵ cells per well.

In one embodiment, the MSC population is counted and initially incubated with the biocompatible matrix at a density of at least 1x10⁴, 2.5x10⁴, 5x10⁴ or 8x10⁴ cells per cm² of culture dishes or plates. In another embodiment, the MSC population is initially incubated with the biocompatible matrix at a density from about 1x10⁴ to about 15x10⁴ cells/cm² of culture dishes or plates, preferably from about 2.5x10⁴ to about 12.5x10⁴ cells/cm², more preferably from about 5x10⁴ to about 8x10⁴ cells/cm². In a preferred embodiment, the MSC population is initially incubated with the biocompatible matrix at a density of about 5x10⁴ cells per cm² of culture dishes or plates.

In one embodiment, the MSC population is counted and initially incubated with the biocompatible matrix at a density of at least 0.25x10⁵, 0.5x10⁵, 1x10⁵ or 1.5x10⁵ cells per cm² of culture dishes or plates. In another embodiment, the MSC population is initially incubated with the biocompatible matrix at a density from about 0.25x10⁵ to about 2.5x10⁵ cells/cm² of culture dishes or plates, preferably from about 0.5x10⁵ to about 2x10⁵ cells/cm², more preferably from about 1x10⁵ to about 1.5x10⁵ cells/cm². In a preferred embodiment, the MSC population is initially incubated with the biocompatible matrix at a density of about 1x10⁵ cells per cm² of culture dishes or plates.

In one embodiment, the incubation of the biocompatible matrix with the mesenchymal stem cells population of the invention is performed in a culture medium as described hereinabove. In some embodiments, the incubation is performed at 37°C in 5% CO₂.

In one embodiment, the composition is cultured once the MSC population contacts the biological matrix. In one embodiment, the composition of the invention is cultured up to confluence of the MSC. In another embodiment, the composition of the invention is cultured up to at least 70, 75, 80, 85, 90, 95, 99, or 100% confluence of MSC.

In another embodiment, the composition of the invention is cultured for at least 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 days after contacting the MSC population and the biological matrix. In another embodiment, the composition of the invention is cultured for more than 30 days. In another embodiment, the composition of the invention is cultured from 3 to 30 days, from 5 to 25 days, from 5 to 20 days, from 5 to 15 days or from 5 to 10 days.

In one embodiment, after culture, the composition of the invention is washed to remove the culture medium. In a particular embodiment, the composition is washed with phosphate buffered saline (PBS).

According to one embodiment, the composition of the invention secretes at most 100 pg/ml of SDF-1α, preferably at most 50, 40, 30, 25, 20, 19, 18, 17, 16, 15, 14, 13, 12, 11, 10, 9, 8, 7, 6, 5, 4, 3, 2 or 1 pg/ml, when the composition is cultured at least 24 hours in hypoxia, preferably at about 0.1% O₂.

According to another embodiment, the composition of the invention secretes at most 100 pg/ml of SDF-1α, preferably at most 50, 40, 30, 25, 20, 19, 18, 17, 16, 15, 14, 13, 12, 11, 10, 9, 8, 7, 6, 5, 4, 3, 2 or 1 pg/ml of SDF-1α, when the composition is cultured at least 24 hours in normoglycemia, preferably at a concentration of glucose of about 1 g/l of glucose.

According to another embodiment, the composition of the invention secretes at most 50 pg/ml of SDF-1α, preferably at most 40, 30, 25, 20, 19, 18, 17, 16, 15, 14, 13, 12, 11, 10, 9, 8, 7, 6, 5, 4, 3, 2 or 1 pg/ml of SDF-1α, when the composition is cultured at least 24 hours in hyperglycemia, preferably at a concentration of glucose of about 4.5 g/l.

In one embodiment, the composition of the invention secretes at least 200 pg/ml of VEGF, preferably at least about 250, 260, 270, 280, 281, 282, 283, 284, 285, 286, 287, 288, 299 or 290 pg/ml of VEGF, when the composition is cultured at least 24 hours in hypoxia, preferably at about 0.1% O₂, and in hyperglycemia, preferably at a concentration of glucose of about 4.5 g/l.

In another embodiment, the composition of the invention secretes at least about 90 pg/ml of VEGF, preferably at least about 95, 100, 105, 110, 111, 112, 113, 114, 115, 116, 117, 118, 119 or 120 pg/ml of VEGF, when the composition is cultured at least 24 hours in physiological oxygen levels, preferably at about 5% O₂, and in hyperglycemia, preferably at a concentration of glucose of about 4.5 g/l.

In another embodiment, the composition of the invention secretes at least about 160 pg/ml of VEGF, preferably at least about 161, 162, 163, 164, 165, 166, 167, 168, 169 or 170 pg/ml of VEGF, when the composition is cultured at least 24 hours in physiological oxygen levels, preferably at about 5% O₂, and in normoglycemia, preferably at a concentration of glucose of about 1 g/l.

In one embodiment, the composition of the invention secretes at most 50 pg/ml of SDF-1α, preferably at most 40, 30, 25, 20, 19, 18, 17, 16, 15, 14, 13, 12, 11, 10, 9, 8, 7, 6, 5, 4, 3, 2 or 1 pg/ml of SDF-1α; and at least about 200 pg/ml of VEGF, preferably at least about 250, 260, 270, 280, 281, 282, 283, 284, 285, 286, 287, 288, 299 or 290 pg/ml of VEGF, when the composition is cultured at least 6 hours in hypoxia, preferably at about 0.1% O₂, and in hyperglycemia, preferably at a concentration of glucose of about 4.5 g/l.

In another embodiment, the composition of the invention secretes at most 50 pg/ml of SDF-1α, preferably at most 40, 30, 25, 20, 19, 18, 17, 16, 15, 14, 13, 12, 11, 10, 9, 8, 7, 6, 5, 4, 3, 2 or 1 pg/ml of SDF-1α; and at least about 90 pg/ml of VEGF, preferably at least about 95, 100, 105, 110, 111, 112, 113, 114, 115, 116, 117, 118, 119 or 120 pg/ml of VEGF, when the composition is cultured at least 6 hours in physiological oxygen levels, preferably at about 5% O₂, and in hyperglycemia, preferably at a concentration of glucose of about 4.5 g/l.

In another embodiment, the composition of the invention secretes at most 100 pg/ml of SDF-1α, preferably at most 50, 40, 30, 25, 20, 19, 18, 17, 16, 15, 14, 13, 12, 11, 10, 9, 8, 7, 6, 5, 4, 3, 2 or 1 pg/ml of SDF-1α; and at least about 160 pg/ml of VEGF, preferably at least about 161, 162, 163, 164, 165, 166, 167, 168, 169 or 170 pg/ml of VEGF, when the composition is cultured at least 24 hours in physiological oxygen levels, preferably at about 5% O₂, and in normoglycemia, preferably at a concentration of glucose of about 1 g/l of glucose.

According to one embodiment, the composition of the invention is cultured up to confluence of the MSC population before measuring the SDF-1α and/or VEGF expression level. In another embodiment, the composition is cultured up to at least about 80, 85, 90, 95, 99, or 100% confluence of the MSC population before measuring the SDF-1α and/or VEGF expression level.

In one embodiment, the composition of the invention is a device (such as, for example, a medical device), a film, a three-dimensional structure, a dressing, a composite graft, a soft tissue substitute, a pharmaceutical composition and the like.

In one embodiment, additional components, being either hormone, protein, nucleic acid, lipid and/or carbohydrate in character, may be added to the composition of the invention in any amount that will allow the added component to be positive or negative effector of adherence, growth, differentiation, proliferation, vascularization, engraftment, and three-dimensional modelling of the MSC population in the composition of the invention. Examples of such components suitable for use in the composition include, but are not limited to naturally occurring, variants or fragments of immune-stimulators, immune-suppressors and/or immune-adjuvants such as cytokines, lymphokines, monokines, stem cell growth factors, lymphotoxins, hematopoietic factors, colony stimulating factors (CSF), interferons (IFN), parathyroid hormone, thyroxine, insulin, proinsulin, relaxin, prorelaxin, follicle stimulating hormone (FSH), thyroid stimulating hormone (TSH), luteinizing hormone (LH), hepatic growth factor, prostaglandin, fibroblast growth factor, prolactin, placental lactogen, OB protein, transforming growth factor (TGF), TGF-α, TGF-β, insulin-like growth factor (IGF), erythropoietin, thrombopoietin, tumor necrosis factor (TNF), TNF-α, TNF-β, mullerian-inhibiting substance, mouse gonadotropin- associated peptide, inhibin, activin, vascular endothelial growth factor, integrin, interleukin (IL), granulocyte-colony stimulating factor (G-CSF), granulocyte macrophage-colony stimulating factor (GM-CSF), interferon-α, interferon-β, interferon-γ, SI factor, IL-1, IL-2, IL-3, IL-4, IL-5, IL-6, IL-7, IL-8, IL-9, IL-10, IL-11, IL-12, IL-13, IL-14, IL-15, IL-16, IL-17, IL-18 IL-21, IL-25, LIF, kit-ligand, FLT-3, angiostatin, thrombospondin, endostatin, LT, corticosteroids, cyclosporin, methotrexate and the like and whether or not any of said components are specific or non-specific. Methods of producing variants are well known in the art, and may include, for example, making conservative amino acid changes, or by mutagenesis and assaying the resulting variant for the required functionality.

In one embodiment, additional components may be present in the composition of the invention in any amount that will allow the added component to provide improved usability or handling characteristics, while not significantly altering the stability or form of the composition of the invention. Examples of such components suitable for use in the composition include, but are not limited to antiseptics, antibiotics, antivirals, antioxidants, anaesthetics, plasticizers, preservatives, cellular growth medium and/or cryoprotectants.

The present invention relates to a method for the preparation of a composition according to claim 13.

The instant disclosure also relates to a method for the preparation of a composition comprising a biocompatible matrix and a mesenchymal stem cells population comprising:
a. isolating mesenchymal stem cells (MSC), preferably adipose stem cells,
b. obtaining a substantially pure MSC population, and
c. incubating the MSC population with a biocompatible matrix.

In the instant disclosure, the method for the preparation of a composition comprising a biocompatible matrix and a mesenchymal stem cells population comprises incubating a substantially pure MSC population with a biocompatible matrix.

In the instant disclosure, the method for the preparation of a composition comprising a biocompatible matrix and a mesenchymal stem cells population, comprises:
a. isolating mesenchymal stem cells (MSC),
b. obtaining a MSC population comprising less than 25% of fibroblasts, and
c. incubating the MSC population with a biocompatible matrix.

In the instant disclosure, the method for the preparation of a composition comprising a biocompatible matrix and a mesenchymal stem cells population comprises incubating a MSC population comprising less than 25% of fibroblasts with a biocompatible matrix.

In the instant disclosure, the method for the preparation of a composition comprising a biocompatible matrix and an adipose stem cells population comprises:
a. isolating adipose stem cells (ASC),
b. obtaining an substantially pure ASC population, and
c. incubating the ASC population with a collagen-containing biocompatible matrix.

In another embodiment, the method for the preparation of a composition comprising a biocompatible matrix and an adipose stem cells population may comprise incubating a substantially pure ASC population with a collagen-containing biocompatible matrix.

In one embodiment, the method for the preparation of a composition comprising a biocompatible matrix and an adipose stem cells population may comprise:
a. isolating adipose stem cells (ASC),
b. obtaining an ASC population comprising less than 25% of fibroblasts, and
c. incubating the ASC population with a collagen-containing biocompatible matrix.

In another embodiment, the method for the preparation of a composition comprising a biocompatible matrix and an adipose stem cells population, may comprise incubating an ASC population comprising less than 25% of fibroblasts with a collagen-containing biocompatible matrix.

Another aspect of the present invention is the composition of the invention as described hereinabove for treating, of for use for treating, soft tissue injury in a subject in need thereof.

In one embodiment, the soft tissue injury may result, for example, from disease, trauma or failure of the tissue to develop normally.

In one embodiment, the soft tissue that can be treated by the composition of the invention is selected from the group comprising skin tissue, muscle tissue, dermal tissue, tendon tissue, ligament tissue, meniscus tissue, cardiac valve tissue, vessel tissue, gastric mucosa, tympanic tissue, amnion and bladder tissue.

In one embodiment, the composition of the invention is for treating, or for use for treating, a skin injury. In another embodiment, the composition of the invention is for treating, or for use for treating, a muscle injury.

In one embodiment, the soft tissue injury that can be treated by the composition is selected from the group comprising skin wounds, skin burns, skin ulcers, muscle diseases (such as, for example, inflammatory muscle diseases, neurogenic muscle diseases, myogenic muscle diseases, muscular dystrophies, congenital myopathies, or myasthenia gravis), hernias, surgical wounds (such as, for example, post-operative wounds), or vascular diseases (such as, for example, peripheral arterial diseases, abdominal aortic aneurysms, carotid diseases, venous diseases, or vascular injuries).

In one embodiment, the composition of the invention is for treating, or for use for treating, a skin wound, a skin burn or a skin ulcer. In a particular embodiment, the composition is for treating, or for use for treating, a skin ulcer due to drepanocytosis. In another embodiment, the composition is for treating, or for use for treating, a skin ulcer due to vasculitis. In another embodiment, the composition is for treating, or for use for treating, a diabetic wound, such as for example a diabetic ulcer. In another embodiment, the composition is for treating, or for use for treating, a radionecrosis. In another embodiment, the composition of the invention is for treating, or for use for treating, a muscle disease.

In one embodiment, the soft injury is an acute wound (i.e. before about 2-4 days post-injury). In another embodiment, the soft injury is a sub-acute wound (i.e. between about 2-4 days to 6 weeks post-injury). In another embodiment, the soft injury is a wound at late stage (i.e. about 6 weeks post-injury). In another embodiment, the soft injury is a chronic wound. In one embodiment, a chronic wound is defined by a failure to achieve complete healing after 3 months. In another embodiment, the soft injury is a non-healing wound.

In another embodiment, the composition of the instant disclosure provides an *ex vivo* model for studying a soft-tissue injury in accordance with the preceding aspects.

In one embodiment, the subject is affected by at least one soft tissue injury as described herein above. In one embodiment, the subject is diabetic. In one embodiment, the subject is diagnosed with diabetes, such as, for example, type I diabetes, type II diabetes, gestational diabetes, latent autoimmune diabetes, type 1.5 diabetes, lipoatrophic diabetes, maturity onset diabetes of the young, neonatal diabetes (e.g. permanent neonatal diabetes or transient neonatal diabetes), prediabetes, steroid-induced diabetes. According to an embodiment, the subject to whom the composition is administered suffered of Type I Diabetes Mellitus or of Type II Diabetes Mellitus.

In one embodiment, the subject was not treated previously with another treatment for soft tissue injury. In another embodiment, the subject previously received at least one treatment for soft tissue injury. Examples of other treatments for treating soft tissue injury include, but are not limited to, wound closure with sutures, staple or adhesive tape or glue, anti-inflammatory drugs, drainage of the infection, surgery, skin autografts, ultrasound therapy, hyperbaric oxygenotherapy, nursing care, topical growth factors application and keratinocyte cell spray.

In one embodiment, the biocompatible matrix of the invention is xenogeneic to the subject to which the composition is administered. A non-limiting example is a porcine biocompatible matrix and a human subject.

In another embodiment, the biocompatible matrix of the invention is allogeneic to the subject to which the composition is administered. A non-limiting example is a human biocompatible matrix and a human subject.

In one embodiment, the MSC population of the invention is xenogeneic to the subject to which the composition is administered, i.e. from a member of a different species. In another embodiment, the MSC population of the invention is allogeneic to the subject to which the composition is administered, i.e. from a non-genetically identical member of the same species. In another embodiment, the MSC population of the invention is syngeneic to the subject to which the composition is administered, i.e. genetically identical or closely related, so as to minimize tissue transplant rejection. Syngeneic MSC population include those that are autogeneic (i.e., from the subject to be treated) and isogeneic (i.e., a genetically identical but different subject, e.g., from an identical twin).

In one embodiment, the biocompatible matrix and the MSC population of the invention are both xenogeneic to the subject to which the composition is administered. In another embodiment, at least one of the biocompatible matrix and the MSC population of the invention is allogeneic to the subject to which the composition is administered. In another embodiment, the biocompatible matrix and the MSC population of the invention are both allogeneic to the subject to which the composition is administered.

In one embodiment, the composition of the instant disclosure may be administered to the subject according to any method known in the art. Examples of methods of administration include, but are not limited to, implantation (such as, for example, surgical implantation), topical application, injection, and transplantation with another tissue.

In one embodiment, the composition of the invention is topically administered to the subject. In another embodiment, the composition of the invention is administered by surgical implantation to the subject. In another embodiment, the composition of the instant disclosure is injected to the subject.

In one embodiment, the composition of the invention is administered to the subject at the soft tissue injury site. In some embodiments, the composition of the invention is configured to the shape and/or size of the tissue to be treated. In some embodiments, the composition of the invention is resized before being administered to the subject to the shape and/or size of the tissue to be treated.

The invention also relates to a method for treating a soft tissue injury, comprising administering a composition as described hereinabove. In one embodiment, the composition is administered topically or by surgical implantation. In one embodiment, the composition is administered at the site of the soft tissue injury.

In one embodiment, the method for treating a soft tissue injury comprises administering the composition of the invention in a subject in need thereof. In a particular embodiment, the method for treating a soft tissue injury comprises administering the composition of the invention in a diabetic subject.

Another object of the present invention is a method for enhancing or improving closure of a wound, such as, for example, a non-healing skin wound. The invention also relates to a method for promoting soft tissue regeneration, such as, for example, dermal regeneration or muscle regeneration.

Another aspect of the invention is a method for promoting or enhancing angiogenesis, preferably at the site of a soft tissue injury. Another aspect of the invention is a method for promoting or enhancing synthesis of granulation tissue, preferably at the site of a soft tissue injury. Still another aspect of the invention is a method for reducing fibrotic scar, preferably at the site of a soft tissue injury.

The invention also relates to a method for reducing soft tissue necrosis, such as, for example, skeletal muscle necrosis.

Another object of the present invention is a method for improving the efficacy of an autograft, such as, for example, a skin autograft, comprising administering a composition of the invention before performing the autograft. In one embodiment, the improvement of the efficacy of an autograft is associated with a reduction of ulcerations (frequency and size).

Another object of the disclosure is a method for screening the effect of a compound on the composition of the invention, wherein said method comprises contacting the compound with the composition of the invention and determining the effect of the compound on the cells present in composition.

Examples of determination of the effect of the compound on the cells present in composition include, but are not limited to, secretion of KGF for epidermal remodeling and VEGF for dermal angiogenesis, both for skin regeneration; secretion of VEGF, IGF-1, HGF, FGF, and TGF-β implied in the activation, proliferation and differentiation of progenitor cells, for skeletal muscular regeneration; and secretion of VEGF in function of the tissue oxygenation.

Another object of the disclosure is a method for screening the effect of a compound on the formation of the film, three-dimensional structure, dressing, composite graft, or soft tissue substitute of the invention, wherein said method comprises contacting the compound with the composition of the invention and determining the effect of the compound on the formation of the film, three-dimensional structure, dressing, composite graft, or soft tissue substitute of the invention.

Examples of determination of the effect of the compound on the formation of the film, three-dimensional structure, dressing, composite graft, or soft tissue substitute of the invention include, but are not limited to, pure stem cells in vitro recolonization of a 3D scaffold; and in vitro stem cells spreading for scaffold surface recovering.

Another object of the disclosure is a method for screening the therapeutic effect of a compound on the soft tissue injury as described here above, wherein said method comprises:
- contacting the compound with a composition of the invention obtained with MSCs from the subject to be treated or a subject having the pathology or condition to be treated, and
- determining the therapeutic effect of the compound on the composition of the invention to determine if the compound can be used for treating a soft tissue injury in a patient in need thereof.

Examples of determination of the therapeutic effect of the compound on the composition of the invention include, but are not limited to, selective growth factors profile secretion in function of the wound tissue, such as KGF for skin epidermal regeneration; and capacity of pure stem cells (obtained from diabetic or non-diabetic subjects) to cure a diabetic skin wound.

Another object of the disclosure is a test or a potency test to determine if the composition of the invention is suitable for being used in the treatment of a soft tissue injury, wherein:
- the composition of the invention is prepared as described above,
- the composition or film, three-dimensional structure, dressing, composite graft, or soft tissue substitute of the invention is analyzed to determine if it presents at least one characteristic of the soft tissue to be treated.

Examples of determination of at least one characteristic of the soft tissue to be treated include, but are not limited to, the increased secretion of VEGF from pure stem cells in a 3D composition in hypoxia.

### BRIEF DESCRIPTION OF THE DRAWINGS

**Figure 1** is a graph showing the quantification of total protein extracted from fascia lata at the different stages of the decellularization method.
**Figure 2** is a set of graphs showing the quantification of the growth factors SDF-1 alpha (A), VEGF (B), bFGF (C) and IGF (D) extracted from fascia lata at the different stages of the decellularization method.
**Figure 3** is a graph showing the quantification of the total DNA extracted from fascia lata before and after the decellularization method.
**Figure 4** is a graph showing the quantification of total protein extracted from dermal tissues at the different stages of the decellularization method.
**Figure 5** is a set of graphs showing the quantification of the growth factors SDF-1 alpha (A), VEGF (B) and bFGF (C) extracted from dermal tissues at the different stages of the decellularization method.
**Figure 6** is a graph showing the quantification of the total DNA extracted from dermal tissues before and after the decellularization method.
**Figure 7** is a graph showing the quantification of total protein extracted from cancellous bone at the different stages of the decellularization method.
**Figure 8** is a set of graphs showing the quantification of the growth factors SDF-1 alpha (A), VEGF (B), bFGF (C) and IGF (D) extracted from cancellous bone at the different stages of the decellularization method.
**Figure 9** is a graph showing the quantification of the total DNA extracted from cancellous bone before and after the decellularization method.
**Figure 10** is a graph showing the quantification of total protein extracted from cortical bone at the different stages of the decellularization method.
**Figure 11** is a set of graphs showing the quantification of the growth factors SDF-1 alpha (A), VEGF (B) and bFGF (C) extracted from cortical bone at the different stages of the decellularization method.
**Figure 12** is a graph showing the quantification of the total DNA extracted from cortical bone before and after the decellularization method.
**Figure 13** is a photograph showing ASC and DF in proliferation medium (A) and in osteogenic differentiation medium (B).
**Figure 14** is a graph showing the cell proliferation of ASC and DF according to the number of passages.
**Figure 15** is a histogram showing the cell survival of ASC and DF in proliferation medium without FBS, at 0.1 or 5% O₂.
**Figure 16** is a set of histograms showing KGF secretion (A), b-FGF secretion (B), IGF-1 secretion (C), and HGF secretion (D) of 5 different ASC/DF dilutions in proliferation medium with 4.5 g/l glucose, at 0.1 or 5% O₂.
**Figure 17** is a set of histograms showing VEGF secretion (A) and SDF-1α secretion (B) of 5 different ASC/DF dilutions in proliferation medium with 4.5 g/l glucose, at 0.1% O₂.
**Figure 18** is a set of histograms showing VEGF secretion (A) and SDF-1α secretion (B) of 5 different ASC/DF dilutions in proliferation medium with 4.5 g/l glucose, at 5% O₂.
**Figure 19** is a histogram showing SDF-1α secretion of 5 different ASC/DF dilutions in proliferation medium with 4.5 g/l glucose, at 21% O₂.
**Figure 20** is a set of histograms showing VEGF secretion (A) and SDF-1α secretion (B) of 5 different ASC/DF dilutions in proliferation medium with 1 g/l glucose, at 0.1% O₂.
**Figure 21** is a set of histograms showing VEGF secretion (A) and SDF-1α secretion (B) of 5 different ASC/DF dilutions in proliferation medium with 1 g/l glucose, at 5% O₂.
**Figure 22** is a histogram showing SDF-1α secretion of 5 different ASC/DF dilutions in proliferation medium with 1 g/l glucose, at 21% O₂.
**Figure 23** is a set of graphs showing VEGF (A), SDF-1α (B), and KGF (C) secretions from AMSC, dermal fibroblasts (FD) and keratinocytes (Kc) at 1 g/l of glucose and at 5% O₂ (light grey) or 0.1% O₂ (dark grey).
**Figure 24** is a set of graphs showing VEGF (A), SDF-1α (B), and KGF (C) secretions from AMSC, dermal fibroblasts (FD) and keratinocytes (Kc) at 5% O₂ and at a concentration of glucose of 1 g/l (light grey) or 4.5 g/l (dark grey).
**Figure 25** is a set of graphs showing VEGF (A), SDF-1α (B), and KGF (C) secretions from AMSC, dermal fibroblasts (FD) and keratinocytes (Kc) in physical conditions (5% O₂ and 1 g/l glucose, light grey) and in diabetic wounds conditions (0.1% O₂ and 4.5 g/l glucose, dark grey).
**Figure 26** is a set of graphs showing VEGF secretions from AMSC (A) and dermal fibroblasts (B), from diabetic or non-diabetic human donors, in 0.1% O₂ and 1 g/l of glucose (0.1% nmglc), in 0.1% O₂ and 4.5 g/l of glucose (0.1% Hglc), in 5% O₂ and 1 g/l of glucose (5% nmglc), and in 5% O₂ and 4.5 g/l of glucose (5% Hglc).
**Figure 27** is a set of graphs showing KGF secretions from AMSC (A) and dermal fibroblasts (B), from diabetic or non-diabetic human donors, in 0.1% O₂ and 1 g/l of glucose (0.1% nmglc), in 0.1% O₂ and 4.5 g/l of glucose (0.1% Hglc), in 5% O₂ and 1 g/l of glucose (5% nmglc), and in 5% O₂ and 4.5 g/l of glucose (5% Hglc).
**Figure 28** is a photograph showing mesenchymal lineage differentiation of ASC: chondrogenic and adipogenic differentiation capacities were demonstrated by alizarin red staining (calcium deposition) (A), alcian blue staining (glycosaminoglycane deposition) (B) and Oil Red (intracellular lipid droplets) (C), respectively (magnification x20).
**Figure 29** is a graph showing spreading (A) and adhesion (B) of ASC on HACM compared to ASC on plastic well plate.
**Figure 30** is a photograph showing histological and macroscopic analyses 1 month and 3 months after implantation of the composite graft (HACM + ASC) and the scaffold alone (HACM, control) in nude rats.
**Figure 31** is a graph showing VEGF secretion by ASC during normoxia (21% O₂) or hypoxia (0.1% O₂).
**Figure 32** is a graph showing vessel density (vessel count/2.56 cm²) of dermis of nude rats 1 month after subcutaneous implantation of HACM + ASC in comparison with HACM alone (p=0.002; five regions of interest were analyzed per slide).
**Figure 33** is a photograph showing clinical evolution of patient 1 suffering of radionecrosis.
**Figure 34** is a photograph showing clinical evolution of patient 1 (A), 2 (B) and 3 (C) before the implantation (1), at the time of implantation (2), and after 22, 4 and 2 months (patient 1, 2 and 3 respectively) (3).
**Figure 35** is a graph showing C-reactive protein (CRP) and fibrinogen concentrations at the time of implantation, and after 3, 13 and 28 days.
**Figure 36** is a photograph showing macrohistology with Masson trichome staining of the wound bed tissue at day 0 (A) and day 56 (B) after implantation in patient 1.
**Figure 37** is a graph showing histomorphometric semi-quantitative analysis of VEGF and factor VIII (A) and of CD3 and CD68 (B) before (light grey) and after implantation (dark grey) in patient 1.
**Figure 38** is a set of photographs showing the wound closure evolution of skin autograft alone (A), skin autograft after composite graft HACM+ASC implantation (B) and skin autograft after HACM implantation alone (C) in the patient 2; at day 0, macroscopically (1) and at dermis tissue level (2), and at the maximum duration of wound closure (28 days, 65 days and 16 days respectively) (3).
**Figure 39** is a graph showing the BM-MSC and ASC survival in hypoxia (0.1% O₂), relative to normoxia (21% O₂).
**Figure 40** is a set of graphs showing VEGF (A), FGF-beta (B), IGF-1 (C), TGF-beta (D) and HGF (E) secretion of BM-MSC and ASC in normoxia (21% O₂) and hypoxia (0.1% O₂). Results are expressed as mean±SEM.
**Figure 41** is a graph showing the cellular spreading of BM-MSC and ASC on the HACM (triangles and squares respectively) in comparison with the cellular spreading on plastic well (straight line) (*p<0.05).
**Figure 42** is a graph showing the cellular growth of BM-MSC and ASC on the HACM (triangles and squares respectively) in comparison with the cellular spreading on plastic well (straight line) (*§p<0.05).
**Figure 43** is a set of graphs angiogenesis (A) and fibrosis thickness (B) of muscular necrosis treated with HACM recellularized with ASC or BM-MSC, or with HACM alone. Results are presented as ratio in comparison to muscular necrosis untreated (sham).
**Figure 44** is a graph showing the angiogenesis of explanted graft, HACM recellularized with ASC or with BM-MSC, or HACM alone, 30 days after implantation (p<0.05).

### EXAMPLES

The present invention is further illustrated by the following examples.

### Example 1: Bioactivity of decellularized matrices

### Material and methods

### Decellularization of human tissues

Human fascia lata and dermal tissues were procured according to the common standards of the European Association of Musculoskeletal Transplantation (EAMST, Vienna, 1997).

Human fascia lata was treated in absolute acetone for up to 24 hours, in ether for 15 hours, then in ethanol 70° for up to 8 hours. Between each step, the tissue was intensely washed in a continuous flow of demineralized water. Human fascia lata was then treated with a combination of NaOH 1N and NaCl for 1 hour, and with H₂O₂ 15% for up to 8 hours. The tissue was further intensely washed with demineralized water for up 68 hours.

Human dermal tissue was treated in ether for 8 hours, then in ethanol 70° for up to 16 hours and washed with demineralized water for 7 hours. Human dermal tissue was then treated with a combination of NaOH 1N and NaCl for 1 hour, followed by a washing with demineralized water for 16 hours, and then treated with H₂O₂ 15% for up to 15 hours. The tissue was further intensely washed with demineralized water for up 16 hours.

Human cancellous bone was procured from tibia and cortical bone from calcaneum.

The treatment of the bone tissues (cancellous and cortical bone) began with a centrifugation to eliminate the marrow and the blood. After the centrifugation, transplants were cut and cleaned with demineralized water. Bone tissues were then treated in acetone for up to 68 hours followed by a washing for 5 hours. Bone tissues were subsequently treated with a combination of NaOH 1N and NaCl for 1 hour, followed by a washing with demineralized water for 3 hours, and then treated with H₂O₂ 15% for up to 15 hours. The tissue was further intensely washed with demineralized water for up 72 hours.

In each stage of the decellularization method, a sample was taken for proteins extraction. Moreover, total DNA was quantified on native tissues, i.e. before decellularization, and on decellularized tissues, i.e. at the end of the decellularization method.

### Protein extraction and purification

Proteins from human fascia lata and dermal tissues were extracted according to the protocol of Wolf et al. (Biomaterials. 2012, 33(10):2916-2925). In brief, 300 mg of tissue were incubated with 5 ml of urea-heparin buffer, pH 7.4 (urea 2M, tris 50 mM, heparin 5 mg/ml, N-ethylmaleimids 10 mM, benzamidine 5 mM and phenylmethylsulfonyl fluoride 1 mM) under stirring for up to 24 hours at a temperature of 4°C. After a centrifugation at a speed of 3000 g for 30 min, the supernatant was removed and treated according the same extraction protocol. The second supernatant was then purified by exclusion chromatography and quantified by ELISA.

Proteins from human bone tissues were extracted according to the protocol of Pietrzal et al. (Radiat Oncol. 2007, 2(1):5). In brief, cancellous and cortical bones were mechanically crushed to obtain a bone powder. 300 mg wet weight of bone powder were incubated with 5 ml of a solution comprising 4M of guanidine and 5 mM of benzamidine for up to 24 hours at a temperature of 4°C. Then 5 ml of a Tris-HCl buffer were added and incubated for up 5 hours at 4°C. After a centrifugation for 10 min, the supernatant was removed purified by exclusion chromatography and quantified by ELISA.

The determination of total protein was carried out with the BCA Assay kit in accordance with supplier's instructions.

For each sample, growth factors levels were quantified by ELISA kits.

### Results

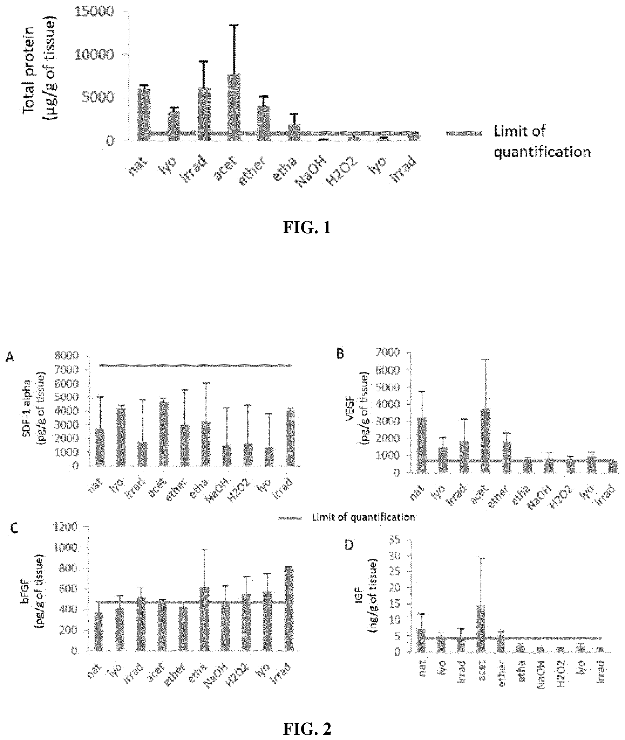

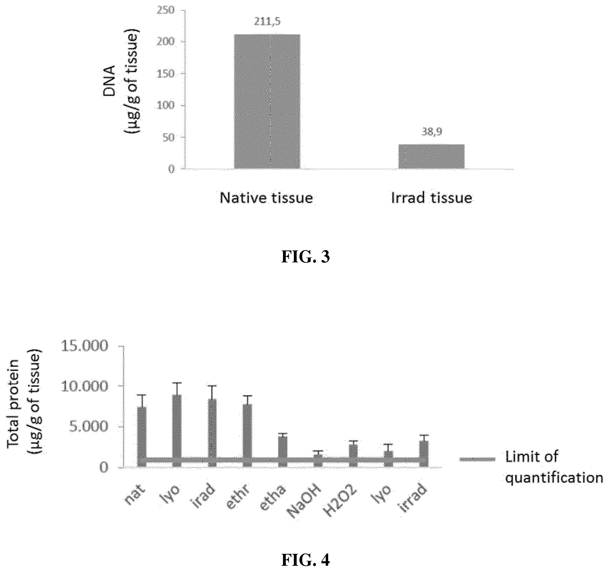

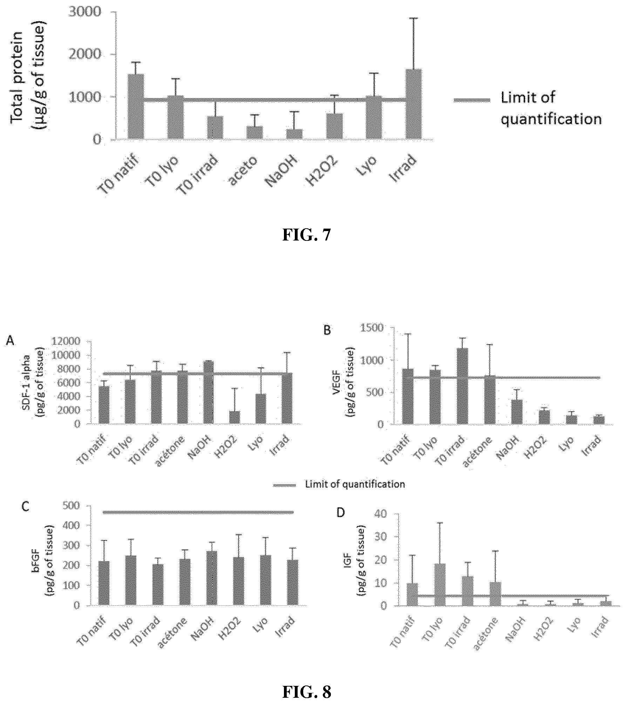

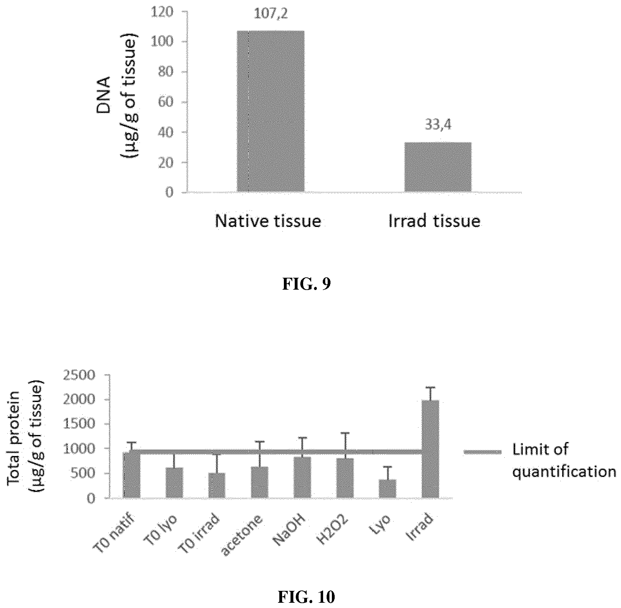

For human fascia lata and dermal tissues, the quantification of total protein shows a decrease of protein levels over decellularization method (Figures 1 and 4). For bone tissues, both cancellous and cortical, total protein decreases at each stage of the treatment, before an increase at the last stage (Figures 7 and 10). This could be due to the gamma irradiation which would promote hydrogen interactions and/or disulfide bonds formation between fragments of degraded proteins.

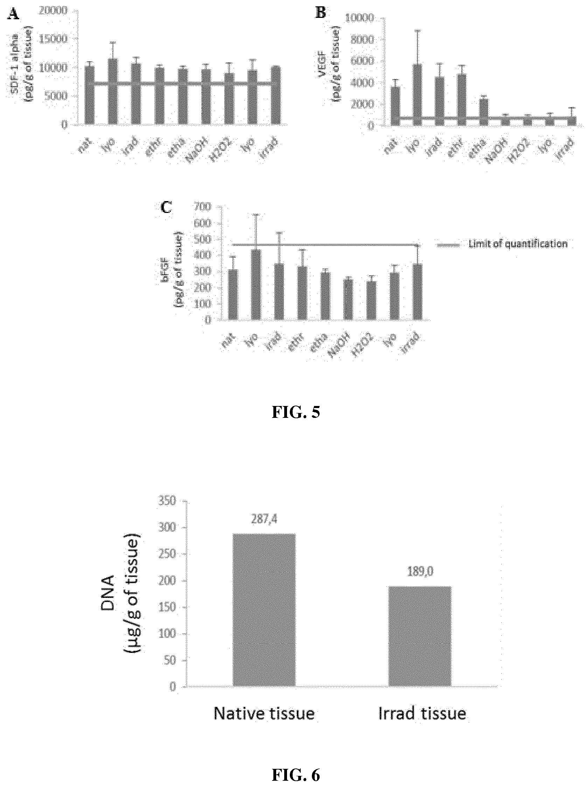

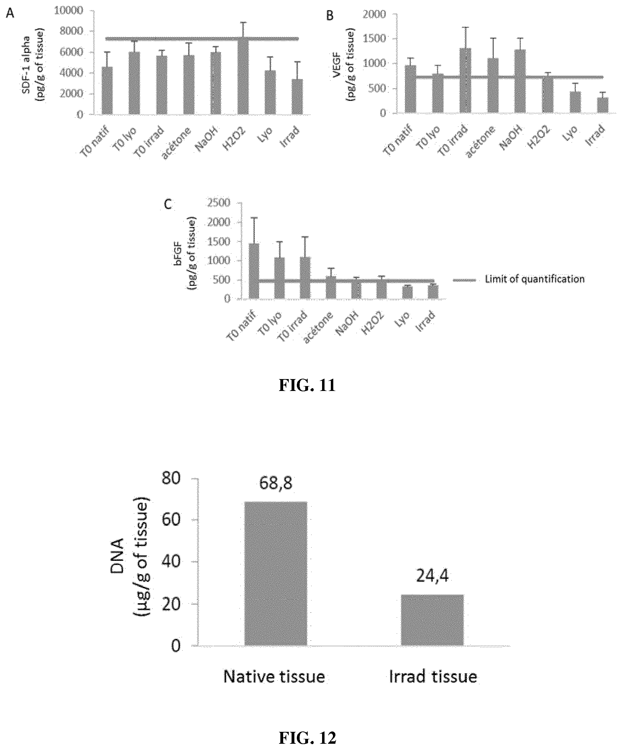

Results of cytokines quantification also indicate a strong decrease of growth factors levels over treatment (Figures 2, 5, 8 and 11). In particular, VEGF and IGF levels strongly decreased at each stage of the decellularization method (Figures 2B and D, 5B, 8B and D, and 11B). No PDGFBB and BMP2 were found in the tissues.

For all tested tissues, DNA quantification also shows a severe decrease of the DNA present in the decellularized matrix in comparison to the DNA in native tissues (Figures 3, 6, 9 and 12). The results indicate an elimination of 82% of DNA content for fascia lata, 35% for dermal tissue, 69% for cancellous bone and 65% for cortical bone.

Taken together, these results show a strong proteins degradation, in particular degradation of cytokines, and DNA elimination. Therefore, decellularized matrices obtained by the method of the invention are inert material and could be administered to patients without fear of a rejection or emergence of a new pathology.

### Example 2: ASC growth factors secretion

### Materials and Methods

This study was performed according to the guidelines of the Belgian Ministry of Health. All procedures were approved by the Ethical Committee of the Medical Faculty (Université Catholique de Louvain) for tissue procurement and clinical study (B40320108280). All materials were obtained from Lonza (Verviers, Switzerland), Sigma-Aldrich (St. Louis, MO, USA), or Invitrogen (Carlsbad, CA, USA) unless otherwise noted.

### ASC and DF isolation and culture

A combined harvesting of human adipose (mean: 7.4 g) and dermal (mean: 1.5 cm²) tissues were performed in 8 patients (Table 1) undergoing elective plastic surgery after informed consent and serologic screening, by lipoaspiration using the Coleman technique, and skin biopsy, respectively. Adipose tissue and skin samples were kept in sterile conditions for a maximum of 60 minutes at 4°C before adipose-derived stem cells (ASC) and dermal fibroblasts (DF) isolation.

**Table 1: Coupled ASC/DF donors characteristics**

| **Donor** | **1** | **2** | **3** | **4** | **5** | **6** | **7** | **8** |
|---|---|---|---|---|---|---|---|---|
| **Age (years)** | 19 | 44 | 40 | 62 | 56 | 46 | 45 | 41 |
| **Sex** | F | F | F | F | F | F | F | F |
| **Clinical indication** | mamm oplasty | abdomin oplasty | mamm oplasty | Lat. Dorsi flap | abdomin oplasty | mamm oplasty | mamm oplasty | mamm oplasty |

The adipose tissue was digested with collagenase (1/2 w/v) in a water bath at 37°C for 60 minutes. Collagenase was inactivated in Dulbecco's modified Eagle medium (DMEM) supplemented with 10% fetal bovine serum. Collected tissue was centrifuged for 10 minutes at 1500 rpm at room temperature. The pellet was suspended in a proliferation medium made up of DMEM supplemented with 10% fetal bovine serum, L-glutamine (2 mM), and antibiotics (100 U/ml penicillin, 100 µg/ml streptomycin, and 1 µl/ml amphotericin B) and filtered through a 500-µm mesh screen. The collected suspension was then seeded in 25 cm² culture flasks with proliferation medium.

DF were isolated by extraction from de-epidermized dermal biopsies, minced in 2 mm x 2 mm fragments and placed in plastic well. Small volume of the proliferation medium was added to avoid detachment from the plastic surface.

After 24 hours of incubation at 37°C and 5% CO₂, the proliferation media were replaced. This initial passage of the primary cells is referred to as passage 0. Dermal pieces were removed from the culture dish when adherent cells were visible on the plastic surface surrounding tissue fragments. Cells were maintained in proliferation medium (changed 2 times/week) up to passage 4, after sequential trypsinizations. Cells from 3 donors were cultivated until passage 15 to study the proliferation profile in standard culture conditions (37°C, 21% O₂, 5% CO₂, 4.5 g/l glucose).

### Membrane marker profile characterization

At passage 4, ASC and DF were characterized for standard cell surface markers (CD44, CD45, CD73, CD90, CD105, stro-1, CD106, CD146, CD166, CD19, CD31, CD11b, CD79α, CD13, HLA-DR, CD14, CD34) [Dominici et al., Cytotherapy. 2006; 8(4):315-317; Bourin et al. Cytotherapy. 2013; 15:641-648] by fluorescence-activated cell sorting (FACScan; BD Biosciences, San Jose, CA).

Briefly, ASC were stained with saturating amounts of monoclonal antibodies: anti-Stro-1, anti-CD90, anti-CD 106, anti-CD105, anti-CD 146, anti-CD 166, anti-CD44, anti-CD 19, anti-CD45 (Human Mesenchymal Stem Cell marker antibody panel, R&D System, Minneapolis, MN, USA), anti-CD44 (PE mouse anti-human CD44, BD Bioscience, Franklin Lakes, NJ, USA), anti-CD73 (FITC mouse anti-human CD73, BD Bioscience), anti-CD31 (FITC, mouse anti-human, Abcam, Cambridge, UK), anti-CDllb (FITC, mouse anti-human, Abcam, Cambridge, UK), anti-CD79α (PE, mouse anti-human, Abcam, Cambridge, UK), anti-CD13 (FITC, mouse anti-human, Abcam, Cambridge, UK), anti-HLA-DR (FITC, mouse anti-human, Abcam, Cambridge, UK), anti-CD14 (FITC, mouse anti-human, Abcam, Cambridge, UK), anti-CD34 (PE, mouse anti-human, Abcam, Cambridge, UK). At least 10,000 gated events were analyzed by flow cytometry with CellquestPro software. Results are expressed in mean fluorescence intensity (MFI), and expressed as percentage of positive cells (threshold: 95% of isotype).

### Differentiation capacity

ASC and DF were tested at passage 4 in specific media to assess the capacity of differentiation toward osteogenic lineage. The differentiation was evaluated by Alizarin red staining after culturing the cell during 3 weeks in specific differentiation medium (proliferation medium supplemented with dexamethasone (1 µM), sodium ascorbate (50 µg/ml), and sodium dihydrophosphate (36 mg/ml) [Qu et al., In Vitro Cell Dev Biol Anim. 2007; 43:95-100]. Osteogenic differentiation was confirmed by staining for calcium phosphate with Alizarin red after formalin fixation. In addition, immunohistochemistry for osteocalcin was performed to confirm the bone phenotype.

### Impact of oxygen tension and fetal bovine serum (FBS) on cell proliferation: EdU assay

Cell proliferation capacity was tested by direct DNA synthesis measurement by 5-ethynyl-2'-deoxyuridine incorporation using Click-iT® EdU Alexa Fluor® 488 Flow Cytometry Assay Kit (Life Technology, Waltham, MA, USA). ASC (n=3) and DF (n=3) were seeded in 21.5 cm² culture dishes at a density of 5000 cells/cm², and cultured for 24 hours in 10% FBS, 21% O₂. Cells proliferation was then stopped by replacing the proliferation medium by the same, without FBS, for 24 hours. The cells were finally placed for 48 hours in the specific conditions: 0.1% O₂, 5% O₂ and 21% O₂ in proliferation medium supplemented with 1% FBS or 5% FBS and EdU (5-ethynyl-2'-deoxyuridine, a nucleoside analog of thymidine and incorporated into DNA during active DNA synthesis) was added. After revelation with Alexa Fluor® 488, positive cells were counted by flow cytometry (FACScan; BD Biosciences, San Jose, CA).

### Growth factor secretion profile

After trypsinization, cells (after passage 3) were counted and 5 progressive dilutions were obtained: 100% ASC + 0% DF; 75% ASC + 25% DF; 50% ASC + 50% DF; 25% ASC + 75% DF; and 0% ASC + 100% DF, and seeded in 12-well culture plates with cells at a density leading to about 80 % to 95 % confluence in triplicate for incubation in hypoxic chambers (Modular Incubator Chamber MIC-101; Billups-Rothenberg, Del Mar, CA, USA) at 0.1% O₂ and 5% O₂, corresponding to highly hypoxic environment and tissular oxygen tension, respectively. The cells were exposed (for each dilution and oxygen tension) to normoglycaemic (1 g/L) or hyperglycaemic (4.5 g/L) proliferation media. After incubation for 24 hours in these controlled conditions; cell culture supernatants were harvested individually and stored at -20°C for further growth factor quantification by enzyme-linked immunosorbent assay (VEGF, HGF, IGF-1, SDF-1α and basic FGF by Quantikine ELISA kit; R&D System, Minneapolis, MN, USA). Cellular viability was assessed immediately after the hypoxic stress by 3-(4,5-dimethylthiazol-2-yl)-5-(3-carboxymethoxyphenyl)-2-(4-sulfophenyl)-2H-tetrazolium solution (MTS; Promega, Leiden, the Netherlands) assay. Hypoxic/glycaemic stress tests and growth factor quantifications were performed in triplicate and duplicate, respectively. Results are expressed in picograms per millimeter.

### Statistical analysis

The one-sample Kolmogorov test and Q-Q plots were used to assess the normal distribution of values. Statistically significant differences between groups (with normal distribution) were tested by paired t-test and one-way analysis of variance with the Bonferroni post hoc test. Statistical tests were performed with PASW 18 (SPSS; IBM, New York, NY, USA); p< 0.05 was considered significant.

### Results

Surface marker profiles do not allow the distinction between the two cell populations (Table 2).

**Table 2: Surface marker characterization of human ASC and DF**

| | **ASC % of positive cells** | **DF % of positive cells** |
|---|---|---|
| *Mesenchymal (stromal) cells markers* | | |
| CD13 | 99.06 | 99.86 |
| CD44 | 95.53 | 99.97 |
| CD73 | 93.78 | 99.86 |
| CD90 | 98.63 | 100.00 |
| CD 105 | 96.86 | 99.78 |
| CD 166 | 60.74 | 96.51 |

| *Bone marrow-derived MSC markers* | | |
|---|---|---|
| CD106 | 5.41 | 2.83 |
| Stro-1 | 4.03 | 5.73 |
| CD 146 | 7.16 | 33.91 |

| *Endothelial cells markers* | | |
|---|---|---|
| CD31 | 5.59 | 5.41 |

| *Hematopoietic lineage markers* | | |
|---|---|---|
| CD14 | 6.75 | 28.27 |
| CD45 | 5.15 | 0.62 |
| CD11b | 5.80 | 8.65 |
| CD34 | 5.53 | 0.54 |

| *Human leukocyte antigens* | | |
|---|---|---|
| HLA-DR | 6.52 | 1.65 |
| CD19 | 4.51 | 2.05 |
| CD79α | 5.10 | 0.37 |

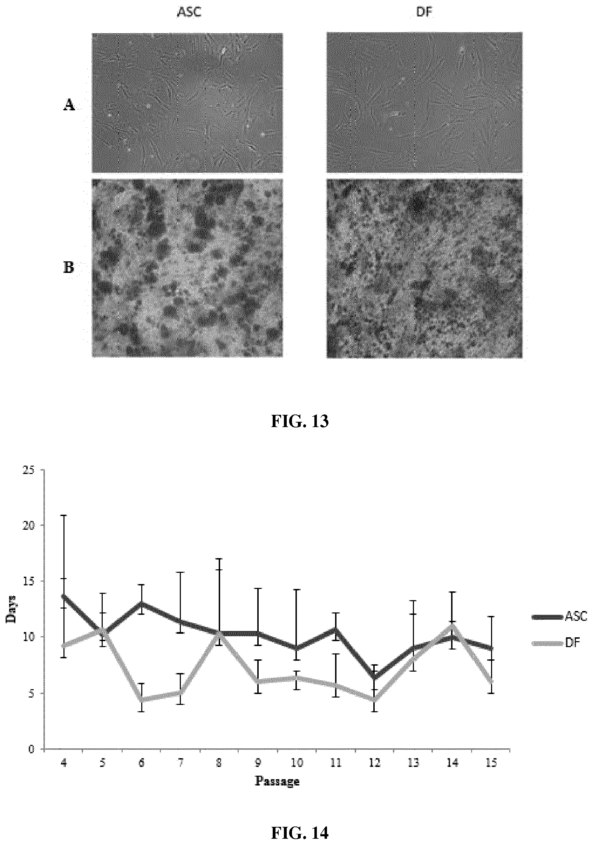

ASC and DF were positive (>90% of positive cells) for mesenchymal cell markers (CD13, CD44, CD73, CD90, CD105, CD166), negative for endothelial (CD31), bone-marrow-derived stromal cells (CD106, Stro-1, CD146) and hematopoietic markers (CD14, CD45, CD11b, CD34), and for HLA-DR, CD79α and CD19. After culture in specific differentiation media (Figure 13), osteogenic differentiation capacitiy was demonstrated for both ASC and DF by Alizarin red staining and osteocalcin immunohistochemistry.

ASC and DF had similar proliferation profile until passage 15 (Figure 14, NS).

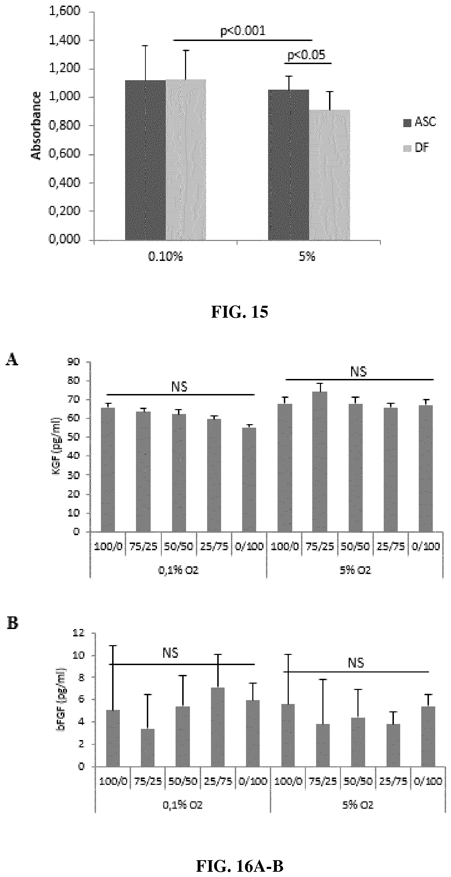

ASC and DF viability was not significantly impacted after 24 hours of culture at 0.1% O₂ and 5% O₂ without FBS (Figure 15). At 5% O₂, DF viability was reduced when compared to ASC (87.04% of ASC survival, p<0.05).

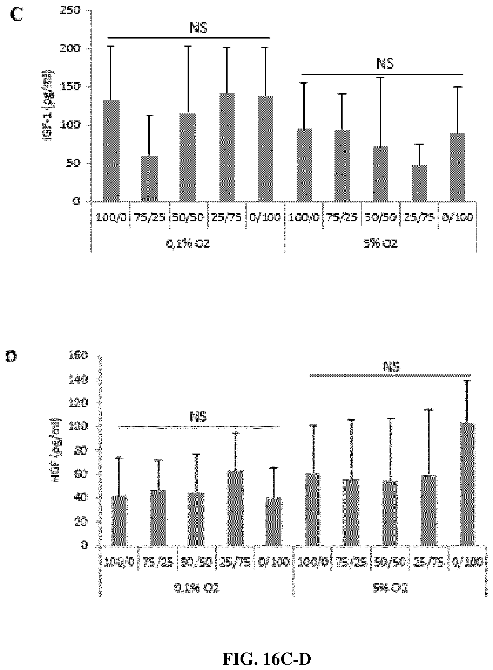

The study of HGF, IGF-1, bFGF and KGF secretion (at 0.1% and 5% O₂, 4.5g/l glucose) from the sequential dilutions of ASC and DF did not demonstrate any significant curve (Figure 16).

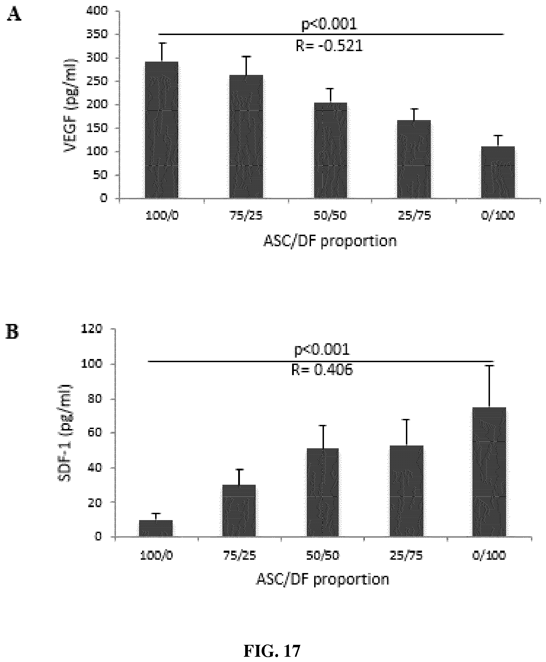

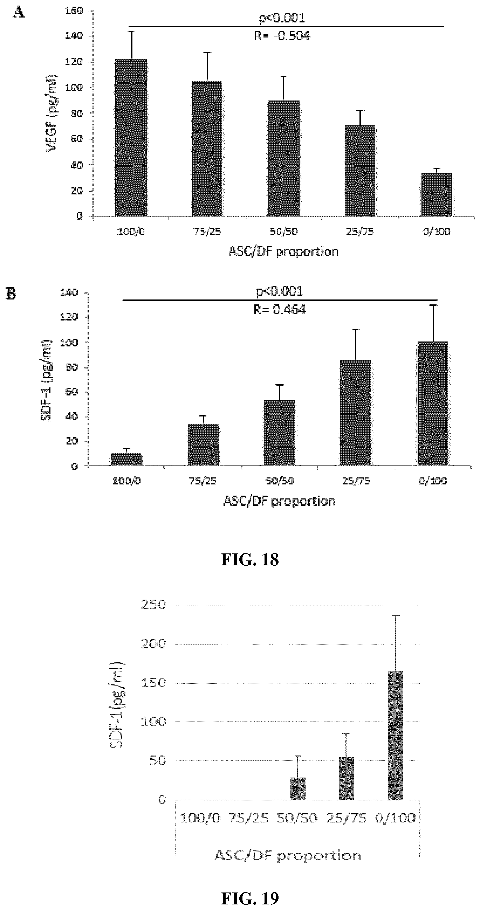

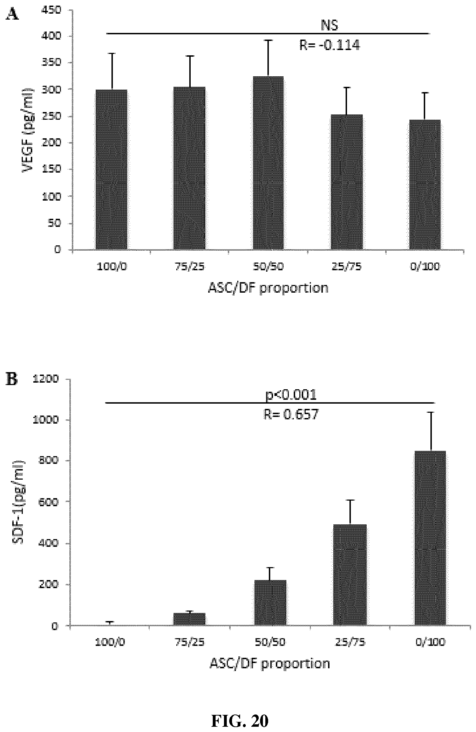

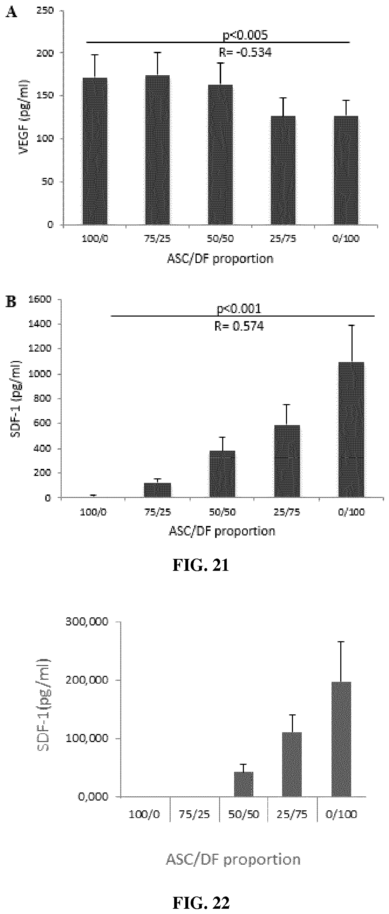

However, for VEGF and SDF-1α, linear regressions following ASC "contamination" by DF were observed. Indeed SDF-1α secretion level decreases with increasing ASC proportion. This result is found in different conditions of oxygen tension (21%, 5% or 0.1%) or of glucose concentrations (1 g/l or 4.5 g/l) (Figures 17 to 22). Moreover, in high glucose culture conditions and at 0.1% O₂ and 5% O₂ VEGF secretion level increases with increasing ASC proportion (Figures 17A and 18A). The same measurements in low glucose conditions demonstrated significant linear regressions for VEGF secretion at 5% O₂ (Figure 20A).

The relations were inversed since DF release higher levels of SDF-1α and VEGF was produced in higher rates by ASC, allowing the measurement of the cell proportion (ASC purity).

### Example 3: ASC comportment in hypoxia and hyperglycemia

### Materiel and methods

This study was performed according to the guidelines of the Belgian Ministry of Health. All procedures were approved by the Ethical Committee of the Medical Faculty (Université Catholique de Louvain).

### ASC, DF and keratinocytes isolation and culture

A combined harvesting of human adipose (mean: 7.4 g) and dermal (mean: 1.5 cm²) tissues were performed in 8 patients (Table 1) undergoing elective plastic surgery after informed consent and serologic screening, by lipoaspiration using the Coleman technique, and skin biopsy, respectively. Adipose tissue and skin samples were kept in sterile conditions for a maximum of 60 minutes at 4°C before adipose-derived stem cells (ASC) and dermal fibroblasts (DF) isolation.

The adipose tissue was digested with collagenase (1/2 w/v) in a water bath at 37°C for 60 minutes. Collagenase was inactivated in Dulbecco's modified Eagle medium (DMEM) supplemented with 10% fetal bovine serum. Collected tissue was centrifuged for 10 minutes at 1500 rpm at room temperature. The pellet was suspended in a proliferation medium made up of DMEM supplemented with 10% fetal bovine serum, L-glutamine (2 mM), and antibiotics (100 U/ml penicillin, 100 µg/ml streptomycin, and 1 µl/ml amphotericin B) and filtered through a 500-µm mesh screen. The collected suspension was then seeded in 25 cm² culture flasks with proliferation medium.

DF were isolated by extraction from de-epidermized dermal biopsies, minced in 2 mm x 2 mm fragments and placed in plastic well. Small volume of the proliferation medium was added to avoid detachment from the plastic surface.

Keratinocytes were purchased from ATCC (PCS-200-011) and cultured with a Keratinocyte Growth Kit (ATCC, PCS-200-040™), in accordance with supplier's instructions.

After 24 hours of incubation at 37°C and 5% CO₂, the proliferation media were replaced. This initial passage of the primary cells is referred to as passage 0. Dermal pieces were removed from the culture dish when adherent cells were visible on the plastic surface surrounding tissue fragments. Cells were maintained in proliferation medium (changed 2 times/week) up to passage 4, after sequential trypsinizations.

### Growth factor secretion profile

After trypsinization, cells (after passage 4) were seeded in 12-well culture plates with cells at a density leading to about 80 % to 95 % confluence in triplicate for incubation in hypoxic chambers (Modular Incubator Chamber MIC-101; Billups-Rothenberg, Del Mar, CA, USA) at 0.1% O₂ and 5% O₂, corresponding to highly hypoxic environment and tissular oxygen tension, respectively. The cells were exposed (for each dilution and oxygen tension) to normoglycaemic (1 g/L) or hyperglycaemic (4.5 g/L) proliferation media. After incubation for 24 hours in these controlled conditions; cell culture supernatants were harvested individually and stored at -20°C for further growth factor quantification by enzyme-linked immunosorbent assay (VEGF, SDF-1α and KGF by Quantikine ELISA kit; R&D System, Minneapolis, MN, USA). Hypoxic/glycaemic stress tests and growth factor quantifications were performed in triplicate and duplicate, respectively. Results are expressed in picograms per millimeter.

### Statistical analysis

The one-sample Kolmogorov test and Q-Q plots were used to assess the normal distribution of values. Statistically significant differences between groups (with normal distribution) were tested by paired t-test and one-way analysis of variance with the Bonferroni post hoc test. Statistical tests were performed with PASW 18 (SPSS; IBM, New York, NY, USA); p< 0.05 was considered significant.

### Results

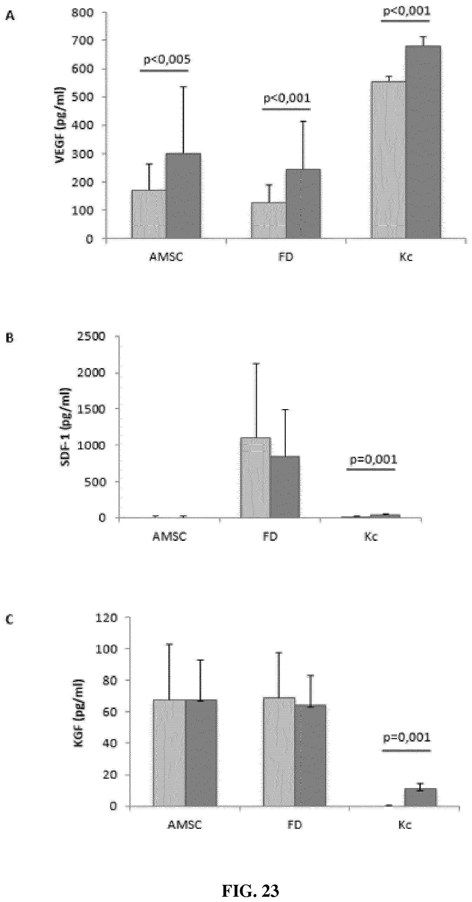

When cells are exposed to hypoxic conditions, i.e at 0.1% O₂, they secrete higher levels of VEGF than in physiological O₂ tension, i.e. at 5% O₂ (Figure 23A). Only keratinocytes show a significant increased expression of SDF-1α and KGF in hypoxia in comparison to normaxia, however levels stay very low (Figures 23B and C).

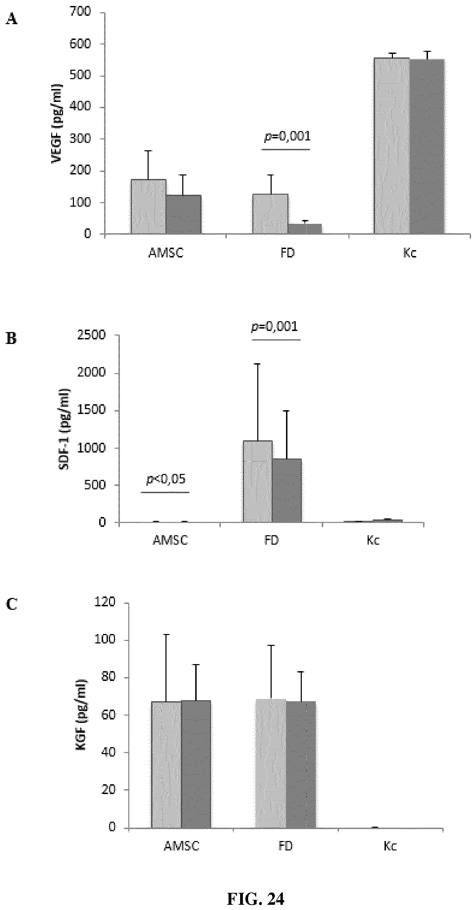

ASC cultured in hyperglycemia, i.e at 4.5 g/l of glucose, show a slight decrease of VEGF secretion in comparison to normoglycemia (1 g/l), while VEGF secretion of fibroblasts drops by more than 70% (Figure 24A). SDF-1α expression levels decrease in both ASC and fibroblasts, and KGF secretion is substantially similar for all cell types (Figures 24B and C).

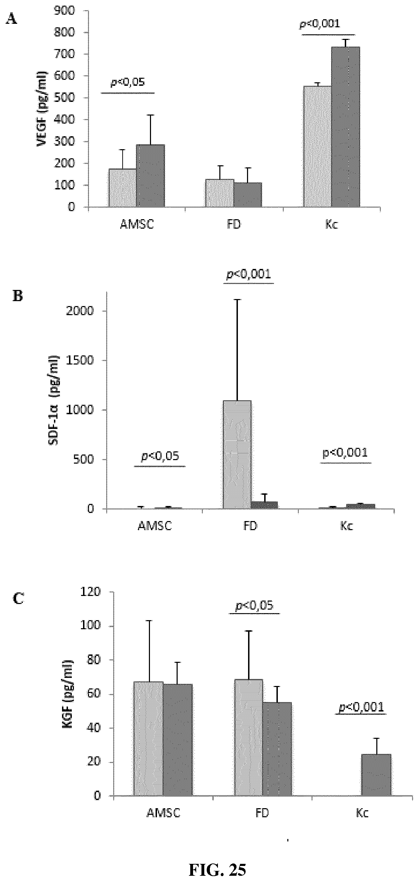

In hypoxia and hyperglycemia, which corresponds to diabetic wounds conditions, ASC secrete significantly more VEGF than in normoxia and normoglycemia (283.94 pg/ml at 0.1% O₂ and 4.5 g/l of glucose, compared to 171.13 pg/ml at 5% O₂ and 1 g/l of glucose; Figure 25A). Fibroblasts, in comparison, secrete lower levels of VEGF in hypoxia and hyperglycemia than in normoxia and normoglycemia.

These results show that ASC secrete more of the growth factor VEGF than fibroblasts in hypoxia, in hyperglycemia and in hypoxia and hyperglycemia. Moreover, diabetic wounds conditions lead to an increased secretion of VEGF from ASC in comparison to physiological conditions. Therefore, ASC can release VEGF to promote neoangiogenesis in a diabetic environment, and, consequently, may promote the repair of diabetic wounds.

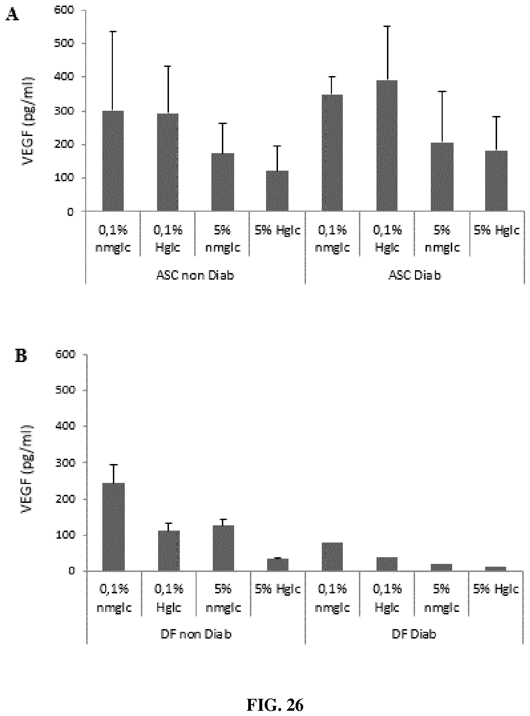

In addition, the secretion profile of VEGF by ASC from non-diabetic human donors or by diabetic human donors are similar (Figure 26A). In particular, VEGF secretion of ASC from diabetic human donors is as high or higher than VEGF secretion of ASC from non-diabetic human donors. In comparison, fibroblasts from diabetic human donors secrete less VEGF than fibroblasts from non-diabetic human donors (Figure 26B).

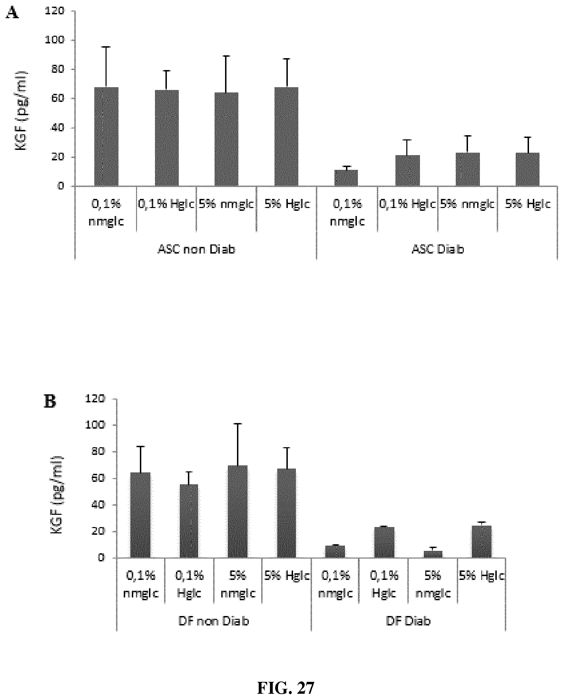

In comparison to VEGF secretion, KGF secretion of ASC from non-diabetic human donors is different to that of ASC from diabetic human donors (Figure 27A). Moreover, similar KGF secretion profiles are found for ASC and for fibroblasts, whether from non-diabetic human donors or from diabetic human donors (Figure 27).

Together, these results show that MSC from a diabetic subject may also release VEGF to promote neoangiogenesis, and, consequently, may promote the repair of a wound, such as a diabetic wound.

Therefore, the MSC population of the invention may derived from a tissue of the subject to be treated.

### Example 4: Dermal regeneration

### Materiel and methods

All procedures were approved by the Ethical Committee of the Medical Faculty (Université Catholique de Louvain) for tissue procurement and clinical study (B40320108280) and by the local Ethics Committee for Animal Care for human and preclinical studies.

### Human ASC

### Isolation and characterization

Human ASC were harvested by lipoaspiration using the Coleman technique (Coleman, Clin Plast Surg. 2001; 28:111-119) in eight patients during elective plastic surgery (abdominal dermolipectomy (n=3) or mammoplasty (n=5), mean of 6.2 g adipose tissue (1.4-14.6 g)) and after informed consent and serology screening.

The adipose tissue was digested with GMP collagenase (0.075 g; Serva Electrophoresis GmbH, Heidelberg, Germany) in a water bath at 37°C for 60 minutes. After tissue digestion, cells were collected after centrifugation and maintained in proliferation medium up to passage 4 (or more for genetic study) after sequential trypsinizations, to obtain a pure ASC population (>90% ASC after 4 passages). The cells were characterized for membrane marker profiles (CD44, CD45, CD73, CD90, CD105, CD34, CD14, CD11b, CD79α, CD19, HLA-DR) (Dominici et al., Cytotherapy 2006;8(4):315-317) by fluorescence-activated cell sorting (FACScan; BD Biosciences, San Jose, CA) and tested in specific media to assess the mesenchymal differentiation capacity (adipogenesis, chondrogenesis, osteogenesis) (Schubert et al., Biomaterials. 2011; 32:8880-8891 ; Cui et al., Biomaterials. 2009; 30:2683-2693).

ASC were also seeded in 12-well culture plates for incubation in hypoxic chambers (Modular Incubator Chamber MIC-101; Billups-Rothenberg, Del Mar, CA, USA) for 72 hours at 0.1% (highly hypoxic environment as seen in necrotic tissues) or 21% O₂ levels (atmospheric normoxia, normal culture conditions) respectively. After incubation, cell culture supernatants were harvested individually and stored at -20°C for VEGF quantification (Quantikine ELISA kit VEGF; R&D System, Minneapolis, MN, USA).

Cellular viability was assessed by 3-(4,5-dimethylthiazol-2-yl)-5-(3-carboxymethoxyphenyl)-2-(4-sulfophenyl)-2H-tetrazolium solution (MTS; Promega, Leiden, the Netherlands) (Vériter et al., Biomaterials 2011;32:5945-5956).

### Ex vivo genetic stability of ASC

Cytogenetic stability was studied by karyotype and fluorescent in situ hybridization (FISH) analyses after different passages to assess the oncogenic safety of the cellular component of the biological dressing. Metaphase chromosomes were obtained from ASC of five donors according to standard protocols. Briefly, cultured cells in the exponential growth phase after passages 1, 4, 10, 12, and 16 were processed for 4 hours with 0.02 µg/ml of Colcemid (Invitrogen, Carlsbad, CA). Harvested cells from the flasks after trypsinization were incubated for 30 minutes at 37°C in hypotonic 0.055 M KCl and fixed in 3:1 methanol:glacial acetic acid. Chromosome harvesting and metaphase slide preparation were performed according to standard procedures (Duhoux et al., PLoS ONE 2011;6:e26311) Eleven to 20 reverse-trypsin-Wright G-banded (GTW) metaphases were analyzed and karyotypes were reported according to the 2013 International System for Human Cytogenetics Nomenclature (ISCN 2013). FISH analysis was performed according to standard protocols (Vériter et al., Biomaterials. 2011; 32:5945-5956) to detect aneuploidy of chromosomes 5, 7, 8, and 18 using TelVysion 5q (SpectrumOrange), CEP7/D7Z1 (SpectrumGreen or SpectrumOrange), CEP8/D8Z2 (SpectrumOrange or SpectrumGreen), and CEP18/D18Z1 (SpectrumGreen) probes (Abbot Molecular, Ottignies/Louvain-la-Neuve, Belgium). Two hundred nuclei were counted for passage 1, passage 4, and passage 16, 120 nuclei were counted for passage 10, and 73 nuclei were counted for passage 12 (the thresholds were calculated following the Beta law with a confidence interval of 99.9%).

### Development of the Biological Dressing

At passage 4, the capacity of ASC to adhere and spread was compared on the human acellular collagen matrix (Dufrane et al., Biomaterials 2008;29:2237-2248) (HACM: freeze-dried decelullarized allogeneic human fascia lata, from the Tissue Bank, University hospital Saint Luc, Brussels, Belgium) and on plastic wells as control. Cell adhesion was assessed by confocal laser scanning microscopy (CLSM), as already described (Bacallao and Stelzer, Methods Cell Biol. 1989; 31:437-452).

Briefly, human ASC were seeded on the processed fascia lata in 24-well at a density of 2×10⁵ cells/well. Every 2 days until day 15 and every 3 days until day 30 after seeding, HACM were washed in phosphate-buffered saline to remove the medium, and then they were immersed in 2 µM calcein acetoxymethyl esters (AM; Molecular Probes Europe BV, Leiden, the Netherlands) for approximately 3 hours to be examined by CLSM. The total cellular covered area, cell perimeter, and shape factor [(area/perimeter²) × 4π] were determined using Scion Image Beta 4.02n acquisition and analysis software (Scion Corporation, Torrance, CA).

To assess the oncologic safety and efficacy of the biological dressing in vivo, pieces (10-mm × 10-mm) of the biological dressing and of the scaffold alone were implanted subcutaneously in nude rats (n=10; Charles River Laboratories International, Wilmington, MA, USA).

Under general anesthesia (isoflurane 3%), a triangular flap was elevated in each paravertebral area to create two subcutaneous pockets, allowing the placement of the grafts (HACM + ASC on the right side and HACM alone on the left side). A thermic lesion (dermal necrosis) was applied on the inner side of each flap to reproduce the hypoxic wound environment. The biologic dressing (HACM + ASC) was implanted with the cells in contact with the burned dermis on the right side, whereas HACM alone was implanted on the left paravertebral area. The flaps were closed with non-absorbable sutures after the placement of five to eight crystals of lithium phthalocyanine (LiPC crystals) to allow further measurement of the post-implantation intra-tissular oxygenation course.

Electron paramagnetic resonance oximetry (EPR spectrometer; Magnettech, Berlin, Germany) was used to follow the intra-tissular PO₂ course and assess the capacity of the composite graft to improve the tissue oxygenation in nude rats. The PO₂ in the injured dermis was studied weekly for up to 4 weeks after implantation under gaseous anesthesia (isoflurane). Results were expressed as percentage of tissue oxygenation at day 6 after implantation.

At 1 month (n=5) or 3 months (n=5) after implantation, complete excision of the graft plus surrounding tissues was performed for macroscopic and histological analysis (hematoxylineosin and Masson trichrome staining for local tumor development and angiogenesis).

### Clinical Application for Dermal Reconstruction

The biological dressing was proposed for three patients with non-healing wounds (Table 3). Peri-umbilical fatty tissue (22 g, 8 g and 21 g, respectively) was harvested (using the Coleman technique under local anesthesia) for ASC isolation and culture in line with Good Manufacturing Practices recommendations.

**Table 3: Patients characteristics**

| | **Age (yr)** | **Sex** | **Wound etiology** | **Wound location/ size** | **Duration of lesions** | **Previous local treatments** | **Previous local evolution** |
|---|---|---|---|---|---|---|---|
| **Patient 1** | 46 | M | Radionecrosis (66 Gy after soft tissue sarcoma resection and pedicled flap dehiscence) | Pretibial (12 cm²) | 13 months | Active dressings, well-conducted nursing care | Deepening of the wound, strong avascular fibrosis |
| **Patient 2** | 21 | M | Drepanocytosis (homozygote, poor control of systemic disease) | 4 supra-malleolar areas (>250 cm²) | 46 months | Active dressings, split-thickness skin autografts x4, hyperbaric oxygenotherapy | Ulcer recurrence after a mean of 23 days after skin graft, hyperalgic lesions |
| **Patient 3** | 41 | F | Vasculitis (systemic lupus erythematosus) | 4 supra-malleolar areas (>200 cm²) | 27 months | Active dressings, ultrasound therapy, split-thickness skin autografts x2 | Complete lysis of skin grafts after 6 weeks and 2 weeks respectively |

At passage 3, ASC were trypsinized, resuspended in DMEM, and loaded on freezedried HACM (corresponding to passage 4). The biologic dressing was obtained when ASC covered more than 90% of the HACM. Finally, the composite graft was rinsed with CMRL (Mediatec, Manassas, VA, USA) and transferred to the operating room for implantation (ASC loaded on the upper side of the HACM).

In these three patients, wound debridement was performed by hydrosurgery before the implantation to ensure a minimally contaminated wound bed. The composite graft was cut to an ideal size and oriented with the cell layer directly in contact with the wound surface and fixed with non-absorbable sutures. Inflammatory parameters were followed (Creactive protein, fibrinogen), as were clinical and histological courses. Vaselinated dressings were applied and changed daily. Biopsies were performed before and after implantation for immunohistochemistry and histomorphometry to study inflammatory reaction, angiogenesis, and tissue remodeling (CD3/CD68, VEGF/factor VIII, and Masson trichrome, respectively).

### Statistical Analysis

The one-sample Kolmogorov-Smirnov test and Q-Q plots were used to assess the normal distribution of values. Statistically significant differences between groups (with normal distribution) were tested by paired t-test and one-way analysis of variance with the Bonferroni post hoc test. Statistical tests were performed with Systat version 8.0 (Cranes Software International, Bangalore, India) or PASW 18 (SPSS; IBM, New York, NY, USA); p< 0.05 was considered significant.

### Results

### Ex vivo and in vivo safety of ASC + HACM

ASC at passage 4th were characterized by mesenchymal stromal cell surface marker profile: CD44+ (>95% of cells), CD73+ (>90%), CD90+ (>95%), CD105+ (>95%), CD45- (<5%), CD34- (<7%), CD14- (<7%), CD11b- (<7%), CD79α- (<7%), CD19-(<5%) and HLA-DR- (<7%) (Table 4), and positive markings for mineralization, hyaline deposition and lipid vacuoles.

**Table 4: Membrane marker phenotype characterized by flow cytometry of non-differentiated ASC**

| **Membrane markers (+)** | **% of positive cells** | **Membrane markers (-)** | **% of positive cells** |
|---|---|---|---|
| **CD44** | >95% | **CD45** | <5% |
| **CD73** | >90% | **CD34** | <7% |
| **CD90** | >95% | **CD14** | <7% |
| **CD105** | >95% | **CD11b** | <7% |
| | | **CD79α** | <7% |
| | | **CD19** | <5% |
| | | **HLA-DR** | <7% |

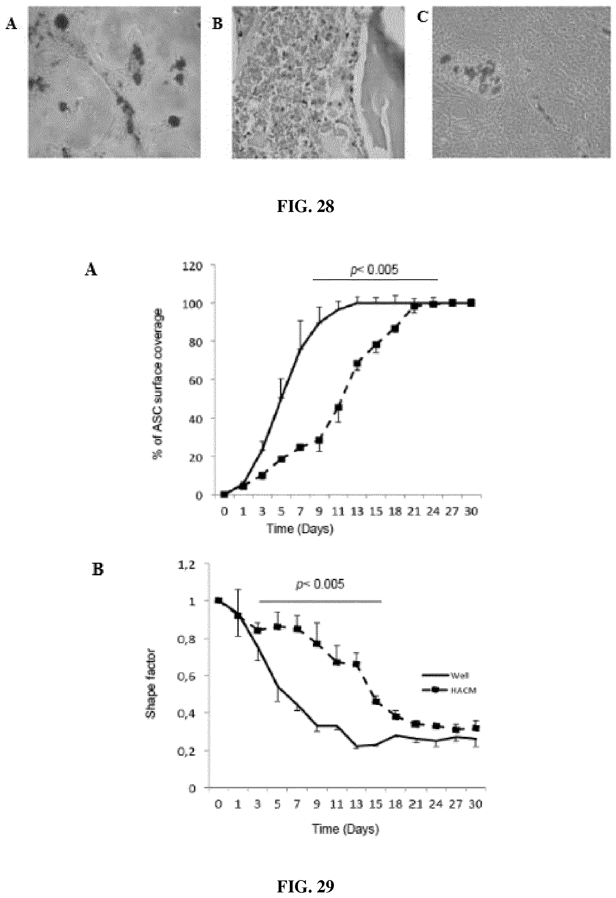

ASC were also characterized in specific media to assess the mesenchymal differentiation capacity (adipogenesis, chondrogenesis, osteogenesis) (Figure 28).

One day after seeding, most ASC appeared round (mean shape factor: 0.93±0.13) on both cellular supports, with a mean of 4.8% surface covering (not significant).

Between days 3 and 18 after seeding, a significantly higher area of cellular expansion was found for plastic wells in comparison with HACM (p<0.005). The complete surface coverage was found on HACM 1 week later than on the plastic well. A similar delay was found for ASC spreading (shape factor) on HACM in comparison with the plastic well (p<0.005) (Figure 29). The covering was delayed but the same cellular growth was reached on the HACM than on plastic well.

The *ex vivo* safety study revealed no clonal structural chromosomal aberrations on the karyotypes of ASC at passage 1, passage 4, and advanced passages (passages 10, 12, or 16). At all passages, borderline tetrasomies (1.5-5.5%) were detected for at least two tested chromosomes by FISH analysis on interphase cells (cut-off: 4.5%). This technique also revealed a clone with suspected monosomy 7 in 15% of interphase cells at passage 16. These aneuploid cells do not seem to have a proliferative advantage because they are not detected in metaphase cells.

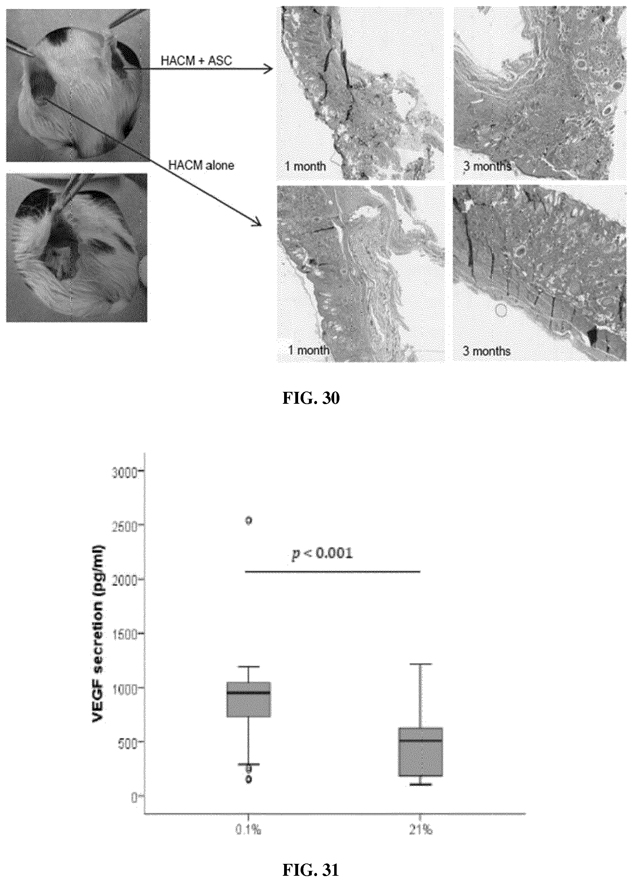

Macroscopic and microscopic analyses did not find any local tumor development in explanted tissue from immunocompromized rats (months 1 and 3 after implantation) (Figure 30).

### Ex vivo and in vivo efficacy of ASC + HACM (Biological Dressing)

Cell viability after 72 hours of incubation at 0.1% O₂ tension was compared with that at 21% O₂ tension (p=0.034). Hypoxia had no deleterious impact on ASC viability and a significantly higher secretion of VEGF was found in hypoxic conditions (0.1% O₂ tension) in comparison with 21% O₂ tension (p<0.001; n=8) (Figure 31).

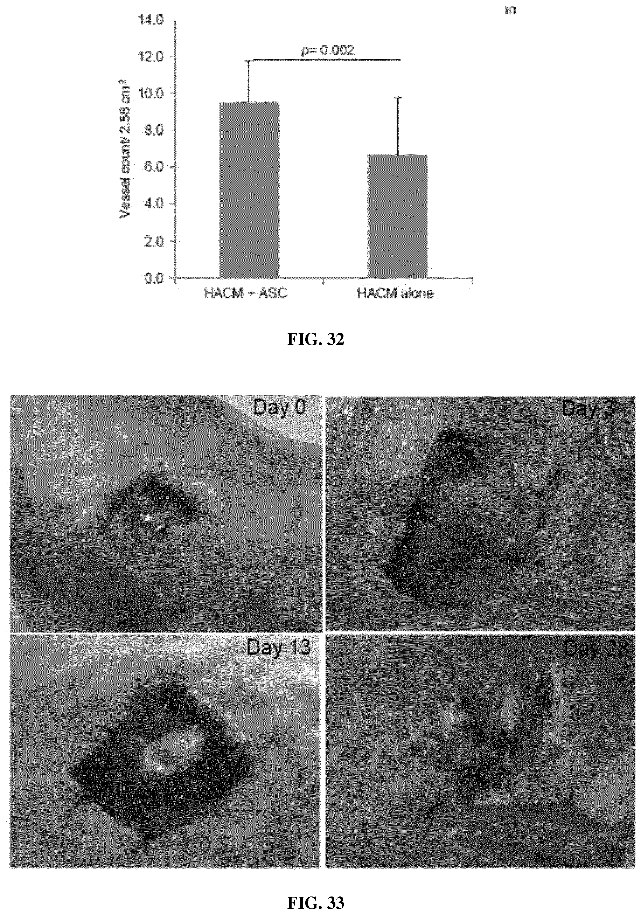

Dermal oxygenation at day 6 (for each individual recipient) was considered as baseline after dermal injury. The ratio of oxygen tension in the deep dermis (after thermic lesion) was significantly higher with HACM plus ASC than with HACM alone (p<0.05 at days 13, 21, and 27 after implantation). Consistently significantly higher vessel density was found in the dermis reconstituted with HACM plus ASC in comparison with the dermis in contact with HACM alone (p=0.002) (Figure 32).

### Clinical application

ASC at the end of the Passage 3th are trypsinized and then seeded on the HACM to obtain the Passage 4th on the collagenic scaffold. The implant was obtained when 90% of the HACM surface was covered with ASC (5.8 × 10⁵ cells per cm²). The complete manufacturing of the dressing, from cell isolation until delivery, was achieved in a mean of 133 days (Table 5).

**Table 5: Timing for the clinical grade production of the biological dressing**

| | **Proliferation Time (Days)** | **Passage Nr** | **Time on HACM (Days) (5-8x10⁵ cells/cm²)** |
|---|---|---|---|
| **Patient 1** | 48 | 4 | 34 |
| **Patient 2** | 97 | 6 | 96 |
| **Patient 3** | 57 | 5 | 68 |

After implantation, initial incorporation of the graft occurred at day 3, followed by progressive resorption of the collagen matrix, which was complete at approximately day 28 after implantation, leaving well-vascularized granulation tissue on the wound bed surface (Figure 33).

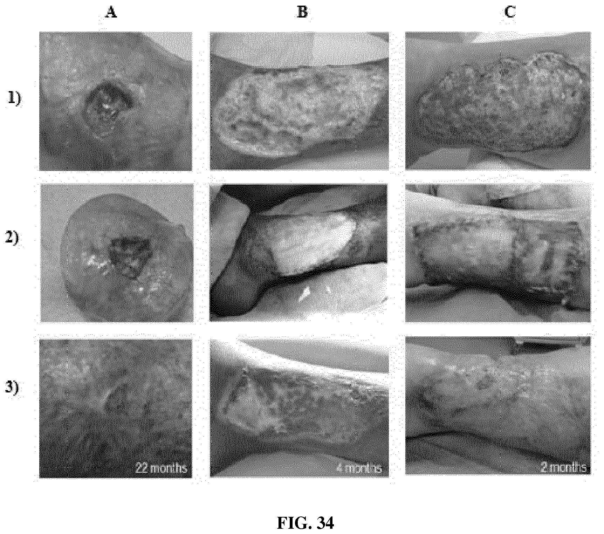

In patient 1, complete wound closure was achieved at day 60 after implantation and has been maintained (more than 22 months). In patients 2 and 3, skin autografts were performed on the vascularized granulation tissue 6 weeks after ASC implantation. The skin autografts were completely integrated after 3 days (first dressing opening) and were associated with pain relief; daily nursing care was discontinued 6 months and 3 months after implantation in patients 2 and 3, respectively (Figure 34). In these two patients recurrence of ulcerations occurred, probably because of the systemic etiologies of wounds (drepanocytosis and vasculitis).

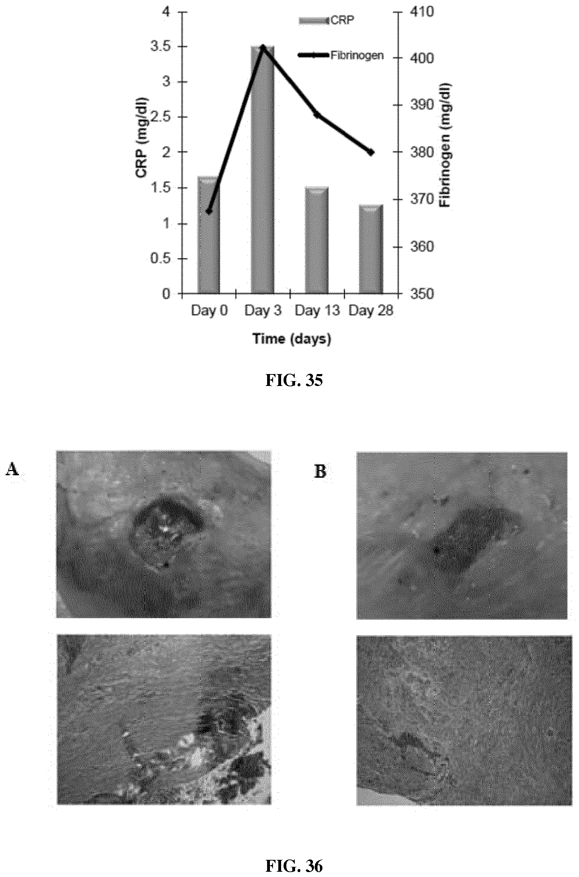

Although a significant increase in C-reactive protein and fibrinogen occurred 1 week after implantation, no chronic inflammation was observed; a decrease to basal values occurred by 1 month after surgery (Figure 35).

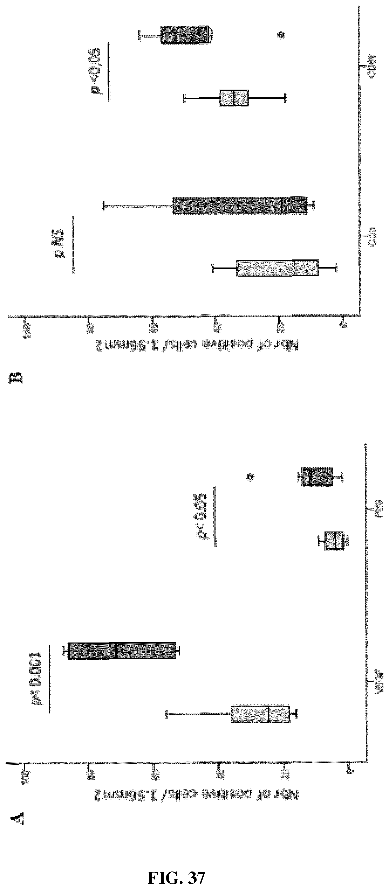

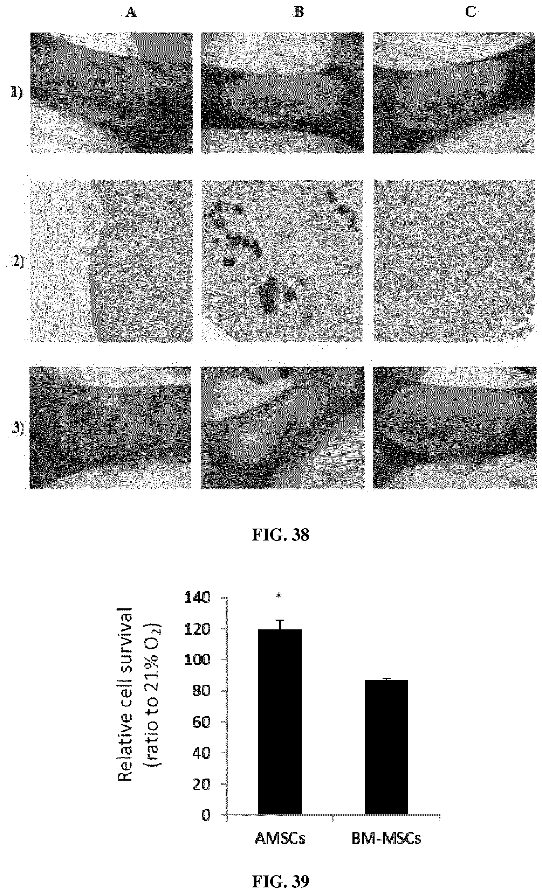

Post-implantation biopsy results revealed well-organized, vascularized tissue in comparison with the strong fibrosis before dressing implantation (Figure 36). Significant increases in VEGF (p<0.001) and factor VIII (p<0.05) were found in tissues after implantation (Figure 37A). Significant macrophagic recruitment was found after implantation (p<0.05), without modification of lymphocyte infiltration (Figure 37B). HACM alone (without cells) did not provide beneficial effects in patient 2 (Figure 38).

Together, these results demonstrate that ASC on HACM can support the restoration of physiology for dermal healing; ASC can survive in a highly hypoxic environment and release VEGF to promote neoangiogenesis, synthesis of granulation tissue, and, consequently, the evolution of healing.

### Example 5: Skeletal muscle regeneration

### Materials and Methods

All materials were obtained from Lonza (Basel, Switzerland), Sigma-Aldrich (St. Louis, MO, USA), or Invitrogen (Carlsbad, CA, USA) unless otherwise specified. All procedures were approved by the local Ethics Committee for Animal Care of the Université Catholique de Louvain (2013/UCL/MD/022).

### BM-MSC and ASC isolation and characterization

Belgium Landrace pigs (female, weight <100 kg, age younger than 6 months old; Rattlerow Seghers Ltd, Lokeren, Belgium) were used as donors for BM-MSC and ASC.

Heparinized BM was harvested and mixed with a double volume of phosphate-buffered saline (PBS). After centrifugation at 450g for 10 min, cells were resuspended at 10⁷ cells/ml, and the cell suspension was layered over a Ficoll-Hypaque column (density 1.077; Lymphoprep, Nycomed, Oslo, Norway) and centrifuged for 30 min at 1250g. The mononuclear cells were collected from the interface and washed in PBS at 450g for 10 min. The cells were placed in culture flasks in Dulbecco's modified Eagle's medium (DMEM) supplemented with 10% heat-inactivated fetal bovine serum (FBS) and antibiotics (Vériter et al., Biomaterials. 2011; 32:5945-5956).

Adipose tissues (mean, 15 g) were washed three times with NaCl 9%, cut in a Petri dish to remove vessels and fibrous connective tissue, and placed in collagenase (0.075 g; Sigma-Aldrich) reconstituted in Hank's balanced salt solution (with calcium and magnesium ions) in a shaking water bath at 37°C with continuous agitation for 60 min. After digestion, the collagenase was inactivated in DMEM supplemented with 10% heat-inactivated FBS, L-glutamine (2 mM), and antibiotics (penicillin 100 U/ml, streptomycin 100 mg/ml). Collected tissue was centrifuged for 10 min at 450g at room temperature. The pellet was then resuspended in proliferation medium (MP) made of DMEM supplemented with 10% FBS and antibiotics (penicillin 100 U/ml and streptomycin 100 mg/ml). After filtration through a 500-mm mesh screen, the tissue was centrifuged for 10 min at 450g at room temperature and then re-suspended in MP media. This initial passage of the primary cells was referred to as passage 0 (P0). After 24 to 48 h of incubation at 37°C in 5% CO₂, the cultures were washed with PBS and maintained in MP media up to passage 4 (P4) and then differentiated in specific media (Schubert et al., Biomaterials. 2011; 32:8880-8891).

Fluorescence-activated cell sorting (at least 10,000 events were analyzed by flow cytometry with CellquestPro software; FACScan, BD Biosciences, Franklin Lakes, NJ, USA) confirmed the mesenchymal stem cell lineage by revealing a positive shift of mean fluorescence intensity for CD44, CD73, CD90, and CD105 antibodies (BD Pharmigen, BD Biosciences) conjugated with phycoerythrin (PE), whereas CD45 antigen expression was negative. In contrast, peripheral blood mononucleated cells (negative control) demonstrated positive staining for CD45 and negative staining for CD44, CD73, CD90, and CD105. At P4, cell differentiations toward adipose, osteogenic, and chondrogenic phenotypes were confirmed by red oil, Alizarin red, and Alcian blue staining, respectively.

### Ex vivo impact of hypoxia on BM-MSC and ASC

ASC and BM-MSC were seeded in 12-well culture plates and incubated in hypoxic chambers (Modular Incubator Chamber MIC-101; Billups-Rothenberg, Del Mar, CA, USA) following a protocol described previously (Schubert et al., Biomaterials. 2011; 32:8880-8891). Cells were incubated for 72 h at 0.1% and 21% O₂ levels in relation to a highly hypoxic environment and atmospheric normoxia, respectively. After 72 h in each condition, cell culture supernatants were harvested individually, centrifuged, and stored at -20°C for subsequent growth factor quantification.

VEGF, IGF-1, FGF, HGF, and TGF-β1 quantification was achieved by enzyme-linked immunosorbent assay (ELISA Quantikine Kit; R&D System, Minneapolis, MN, USA). Samples were not diluted and the supplier's instructions were followed. Optical density of each well was measured by a Multiskan EX Labsystems spectrophotometer (Thermo Scientific, Breda, the Netherlands) set at 450 nm with a correction wavelength set at 690 nm. The growth factor releases were expressed by a ratio between hypoxia and normoxia.

Cellular viability was also assessed under normoxic and hypoxic conditions by an MTS cell proliferation assay (MTS; Promega, Leiden, the Netherlands) as described previously (Schubert et al., Biomaterials. 2011; 32:8880-8891). After incubation, the optical density of each well was measured by a Multiskan EX Labsystems spectrophotometer (Thermo Scientific) set at 450 nm with a correction wavelength set at 690 nm.

### Human acellular collagen matrix (HACM)

Fascia lata from selected donors were procured according to European and Belgian legislation regarding human body materials after human tissue donor screening based on clinical history, serological tests, and microbiological testing. The human fascia lata tendon was prepared as described by using a process developed by the Tissue Bank of the University Hospital Saint-Luc (Brussels, Belgium) to obtain an HACM containing no chemical residues (acetone/H₂O2) and containing less than 5% of residual moisture. It was sterilized by gamma irradiation at 25,000 Gy (Sterigenics, Fleurus, Belgium). Allografts were then stored at room temperature (Dufrane et al., Biomaterials. 2008, 29: 2237-2248).

The decellularization process (of the HACM) was first assessed by 40,6-diamidino-2-phenylindole (DAPI; 1 µg/ml) staining (Abbot Molecular Inc. USA) on paraformaldehydefixed (4%) slices from native (n=4) and processed HACM (n=4) from separate donors. Tissue sections were observed using a fluorescence microscope (Zeiss, Zaventem, Belgium) with Infinity camera and Delta Pix viewer program. Second, DNA isolation was performed with QIAamp kit® DNA Mini Kit (Qiagen, Hilden, Germany) on equal volume paraffin samples from a frozen native matrix (n=4) and a post-processed matrix (n=4). The DNA concentration was measured by fluorometry with a Qubit™ fluorometer (Invitrogen) with a wavelength set at 260 nm. Results were expressed in µg/ml of DNA isolation solution.

### Ex vivo adhesion and spreading of MSC on the HACM

At the end of passage 3 (P3), the cellular capacity of MSC to spread on the HACM was compared with that of cells cultured on plastic wells (as control). The cellular adhesion on the scaffold was assessed by confocal laser scanning microscopy (CLSM) as already described (Dufrane et al., Biomaterials. 2002, 23: 2979-2988).

Cellular adhesion and spreading (on plastic well and HACM) were studied by CLSM between day 1 and day 30 after seeding. Both MSC were seeded on HACM and onto 24-well clusters at a density of 2×10⁵ cells/well. After seeding, at day 1 to day 30, composite grafts were removed from the culture medium, washed in PBS to remove the medium, and immersed in calcein acetoxymethyl esters (AM) 2 mm (Molecular Probes Europe BV, Leiden, Holland) for approximately 3 h. Then, grafts (cells plus HACM) were attached to a slide using cyanoacrylate glue and examined in the CLSM (Bio-Rad MRC 1024) using × 10 air lens and the 488-nm excitation wavelength line from an argon ion laser. Living cells were distinguished by the presence of ubiquitous intracellular esterase activity determined by the enzymatic conversion of the virtually nonfluorescent cell-permeable calcein AM to the intensely fluorescent calcein. Cell proliferation in the plastic well was observed on the same.

One day before day 30, media was extracted. Wells were rinsed with PBS and immersed in calcein AM. The total cell area and the cell perimeter were determined using Scion Image Beta 4.02 acquisition and analysis software. Because the spreading induced changes in cell shape, the shape factor [(area/perimeter²)×4π] was also calculated as a function of substrate and time.

### Mechanical properties of the composite grafts (HACM plus MSC)

The impact of *ex vivo* recellularization of the HACM by MSC was mechanically assessed. Uniaxial mechanical resistance test was performed on triplicate samples of native human fascia lata (n=5), rehydrated freeze-dried HACM (n=4), HACM alone (without MSC) incubated 4 weeks in culture media (n=4), and recellularized HACM with MSC (after 4 weeks of incubation; n=4). Mechanical testing was performed using an Instron traction system with Instron bluehill software (Model 5600; Instron, Canton, MA, USA) with a load-to-failure test at an elongation rate of 4 mm x min⁻¹. Distance between the two grips was 26.5 mm for each test. Samples were cut to a constant length of 45 mm and a width of 15 mm. The thickness of each sample was recorded. The load-elongation behavior of the matrices and failure modes were recorded. The structural properties of the matrices were represented by stiffness (Nm x m⁻¹) and ultimate load (N). Stiffness (k) was calculated as k = ΔF/ΔL, where F is the force applied on the body and L is the displacement produced by the force along the same degree of freedom (for instance, the change in length of a stretched spring). These parameters were compared between each experimental group.

### In vivo efficacy of the composite graft HACM plus MSC

Two experimental models of skeletal muscular defect were developed for nude rats (n=20; Charles River Laboratories International, Wilmington, MA, USA) weighing 200-300 g (5-8 weeks old). Anesthesia was induced and maintained by isoflurane (Abbvie, Wavre, Belgium) inhalation to perform a longitudinal abdominal incision to expose the skeletal musculature of the abdominal wall.

### 1. Electrocoagulation muscular defect

In each animal (n=6), four necrosis areas of 16 mm² were created by electrocoagulation on the abdominal parietal muscle. The electrocoagulated defects were covered by: MSC plus HACM (two zones, cells in direct contact the necrosis area); HACM alone (one zone); and sham (no graft, one zone). The implants were directly placed in close contact with the defect by four 5.0 Prolene® stitches. Animals received either composite grafts made of HACM plus ASC (n=3) or BM-MSC (n=3). The composite grafts were implanted after a period of 21 to 28 days of MSC incubation on the HACM.

### 2. Full-thickness muscular defect of abdominal wall

A full-thickness defect (1.5 x 2.5 cm²) was performed in the abdominal muscular wall (n=14 nude rats), leading to the exposition of the internal organs. The defects were treated by composite graft (HACM plus ASC, n=4; HACM plus BM-MSC, n=4) or HACM alone (n=6).

In both models, the skin was closed using a nonresorbable suture to cover the implantation site. Nude rats were killed on postoperative day 30 by intracardiac injection of T61 (Intervet, Boxmeer, the Netherlands) under general anesthesia. Graft explantation was then performed and implants were processed for histomorphometry.

Graft integration was macroscopically assessed for inflammatory reaction, graft integration, tissue remodeling, adhesions to internal organs, and hernia occurrence in the full-thickness defect model.

Histomorphometry analysis was performed at 4 weeks after implantation to assess angiogenesis and tissue remodeling. Implants were immediately fixed overnight in 4% formaldehyde and paraffin-embedded. Serial sections (5 µm thickness) were mounted on glass and dried for 12 h at 37°C. Hematoxylin and eosin staining and Masson trichrome staining were performed to assess the vascular proliferation and remodeling process. Additionally, muscular recolonization was studied by immunostaining for dystrophin (diluted at 1:450 and revealed by the EnVision anti-rabbit monoclonal antibody; Abcam, Cambridge, UK).

Tissue remodeling was histomorphologically quantified (in the model of electrocoagulation) by measuring the distance between the native intact muscle and the implant (HACM or skin for HACM without or with MSC and sham, respectively) after staining with Masson at x12.5 magnification within a standard micrometer scale. A minimum of five regions of interest (ROIs) were analyzed on each slide. Tissue remodeling was calculated by a ratio between the thickness found with HACM (alone or with MSC) and sham. In the abdominal wall defect model, dystrophin staining was performed to study muscular recolonization of the implant.

Vascular density was studied by counting the vessels at x25 magnification within a standard grid representing a surface of 0.16 mm² on Masson trichrome slides. A minimum of five ROIs were analyzed on each slide.

### Statistical analysis

The one-sample Kolmogorov-Smirnov test and QQ-plots were used to ensure the normal distribution of values. Results were expressed as means±SD unless otherwise mentioned. Statistically significant differences between experimental groups were tested using Student t-test or one-way analysis of variance with a Bonferroni post hoc test. The statistical tests were performed with PASW 18 (SPSS; Westlands Centre, Quarry Bay, Hong Kong). Differences were considered to be significant at p<0.05.

### Results

### Impact of hypoxia on MSC growth factor release for muscular regeneration

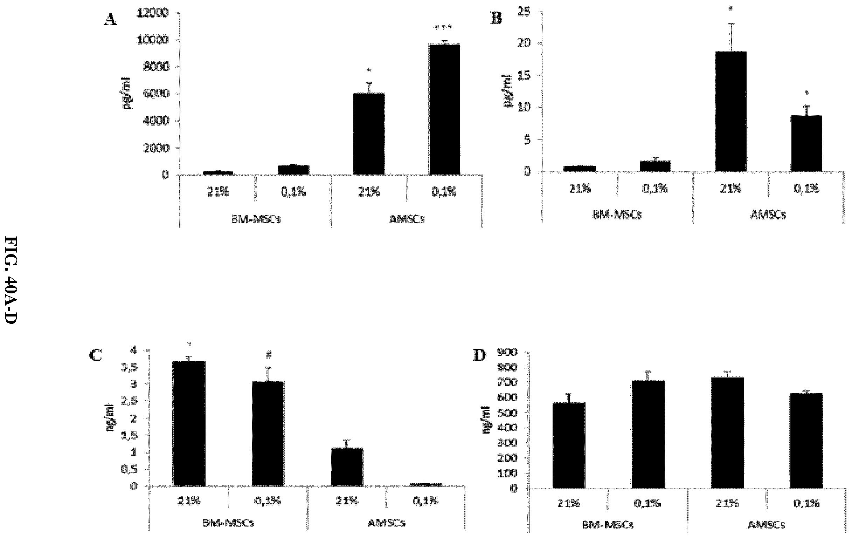

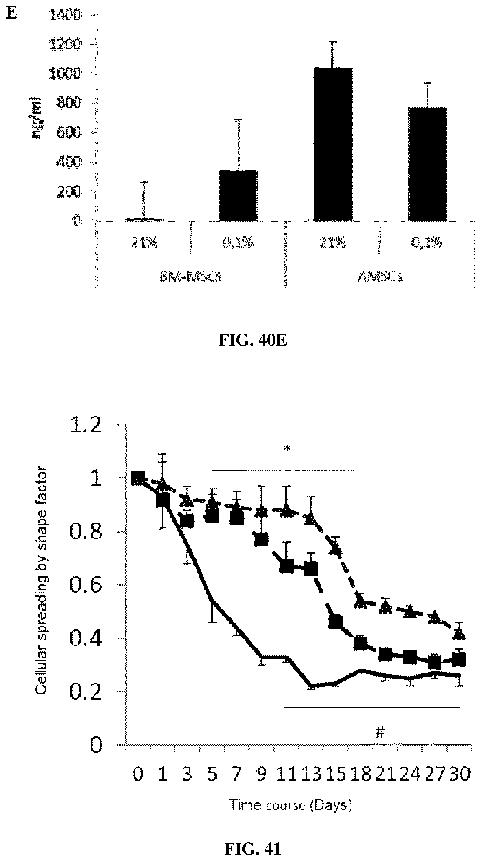

In hypoxia (in comparison with normoxia), a significantly better survival rate was found for ASC in comparison with BM-MSC with 119.5±6.1% vs. 86.8±1.5% of cellular viability, respectively (p<0.05; Figure 39). Significantly higher releases of FGF and VEGF were obtained by AMSC in comparison with BM-MSC at 0.1% and 21% O₂ (p<0.05) (Figure 40, A and B). In addition, hypoxia improved the VEGF release for ASC (+37%; p<0.05) without any impact on BM-MSC.

A significantly lower amount of IGF was secreted by ASC in comparison with BM-MSC in hypoxia as well as normoxia (p<0.005). No significant impact of oxygen tension on TGF and HGF release was found for both MSC (Figure 40, D and E).

### MSC adhesion and spreading on the HACM

HACM decellularization was first confirmed by the detection of rare fluorescent nuclei after DAPI staining on processed matrices in comparison with native matrices (data not shown). No DNA detection was measured by Qubit fluorometer (<0.01 µg/ml in comparison with a mean of 1.45 µg/ml found in four independent native fascia lata).

Optimal spreading (with cellular elongation by a shape factor near 0) on the plastic well was observed on day 13 for both ASC and BM-MSC (0.22±0.001). A significant delay was observed to obtain maximal cellular spreading on the HACM after 27 and 30 days for ASC (0.31±0.03) and BM-MSC (0.48±0.02), respectively. Significantly lower MSC spreading was found between days 5 and 18 (in comparison with plastic well; p<0.05; Figure 41). In addition, a significant difference in MSC spreading (ASC versus BM-MSC) was found between 11 and 30 days after seeding on the HACM (p<0.05; Figure 41).

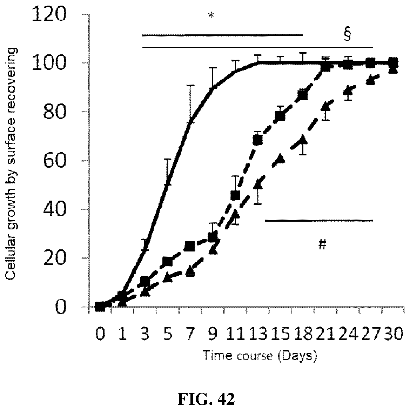

The total recovery of the plastic well was obtained within 2 weeks after incubation with both MSC origins, which was delayed on HACM at days 21 and 30 for ASC and BM-MSC, respectively (p<0.05). The percentage of HACM recovery area was significantly lower in comparison with that of plastic well recovery between day 3 and day 18 and between day 3 and day 27 for both ASC and BM-MSC, respectively (p<0.05). A significant delay of HACM recovery was obtained with BM-MSC in comparison with ASC between days 13 and 28 after incubation (p<0.05) (Figure 42).

### In vivo efficacy of the composite graft HACM plus MSC

### 1. Electrocoagulation muscular defect

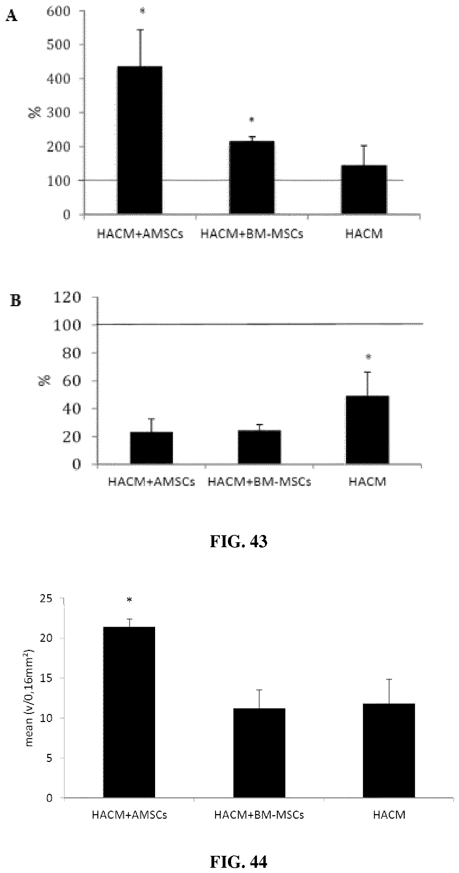

At 4 weeks after implantation, the angiogenesis was significantly higher in areas treated by ASC and HACM in comparison with BM-MSC and HACM and with HACM alone (434±108% vs. 215±13% vs. 144±57% ratio to sham; p<0.001) (Figure 43A).

After 4 weeks, better integration of the skeletal muscle was macroscopically found with the composite graft (HACM plus MSC) in comparison with HACM alone as confirmed by hematoxylin and eosin and Masson trichrome, with significantly lower residual fibrosis (ratio in comparison with sham) for ASC or BM-MSC plus HACM in comparison with HACM alone (23±9% and 24±10% vs. 49±17%, respectively; p<0.05). No difference was found between ASC and BM-MSC (Figure 43B).

### 2. Full-thickness muscular defect of abdominal wall

No abdominal hernia incidence was observed without any intra-abdominal adhesions with abdominal organs with HACM alone or supplemented with MSC. Although some positive dystrophin cells were detected near the suture edge for each recipient with HACM and MSC, no positive cells were found in the core of the abdominal implant. At 4 weeks after implantation, angiogenesis significantly improved in the implant made of ASC and HACM in comparison with BM-MSC plus HACM and HACM alone (21.4±1.0 vs. 11.2±2.3 vs. 11.8±3.1 vessels/grid; p<0.05) (Figure 44).

A composite graft made of ASC demonstrated the capacity of adipose stem cells to survive under *ex vivo* hypoxia, the capacity to obtain optimal cellular delivery by a decellularized collagen matrix scaffold, the capacity to improve the release of proangiogenic factors by an oxygen-sensitive mechanism, the capacity of in vivo vascular recruitment during the early stress phase after transplantation, and, finally, the capacity to reduce the fibrotic scar in comparison with a cell-free scaffold. All these properties could promote skeletal muscle regeneration.

Moreover, these results demonstrated that ASC exert better *ex vivo* and *in vivo* proangiogenic actions with precise control in hypoxia when BM-MSC exert specific anti-fibrotic actions. The HACM is an ideal scaffold for MSC adhesion, spreading, and cell delivery, which remain mechanical requirements for skeletal muscular necrosis and critical size defects.

## Claims

1. A composition comprising a biocompatible matrix and a mesenchymal stem cells population, wherein said mesenchymal stem cells are derived from adipose tissue, wherein said mesenchymal stem cells population is substantially pure, wherein said mesenchymal stem cells are undifferentiated and wherein said mesenchymal stem cells population comprises less than 25% of fibroblasts.

2. The composition according to claim **1**, wherein said mesenchymal stem cells are derived from subcutaneous adipose tissue.

3. The composition according to any one of claims **1** or **2**, wherein said biocompatible matrix is acellular.

4. The composition according to any one of claims **1** to **3**, wherein said biocompatible matrix comprises collagen.

5. The composition according to any one of claims **1** to **4**, wherein said biocompatible matrix is human, porcine, bovine or equine.

6. The composition according to any one of claims **1** to **5**, wherein mesenchymal stem cells originate from the subject to be treated.

7. The composition according to any one of claims **1** to **6**, for use for treating a soft tissue injury in a subject in need thereof.

8. The composition for use according to claim **7**, wherein said soft tissue is selected from the group comprising skin tissue, muscle tissue, dermal tissue, tendon tissue, ligament tissue, meniscus tissue and bladder tissue.

9. The composition for use according to claim **7** or **8**, wherein said soft tissue injury is an acute or a chronic wound.

10. The composition for use according to any one of claims **7** to **9**, wherein said soft tissue injury is selected from the group comprising tears or ruptures of soft tissue, skin wounds, skin burns, skin ulcers, surgical wounds, vascular diseases, muscle disease, hernias and radiation wounds.

11. The composition for use according to claim **10**, wherein said skin wound is a diabetic wound.

12. The composition for use according to any one of claims **7** to **11**, wherein said composition is administered topically or by surgical implantation.

13. A method for the preparation of a composition comprising a biocompatible matrix and a mesenchymal stem cells population, comprising incubating a substantially pure mesenchymal stem cells population with a biocompatible matrix, wherein said mesenchymal stem cells are derived from adipose tissue, wherein said mesenchymal stem cells are undifferentiated and wherein said mesenchymal stem cells population comprises less than 25% of fibroblasts.

## Patentansprüche

1. Zusammensetzung, umfassend eine biokompatible Matrix und eine Population von mesenchymalen Stammzellen, wobei die mesenchymalen Stammzellen von Fettgewebe abgeleitet sind, wobei die Population von mesenchymalen Stammzellen im Wesentlichen rein ist, wobei die mesenchymalen Stammzellen undifferenziert sind und wobei die Population von mesenchymalen Stammzellen weniger als 25 % Fibroblasten umfasst.

2. Zusammensetzung nach Anspruch **1**, wobei die mesenchymalen Stammzellen von subkutanem Fettgewebe abgeleitet sind.

3. Zusammensetzung nach einem der Ansprüche **1** oder **2**, wobei die biokompatible Matrix azellulär ist.

4. Zusammensetzung nach einem der Ansprüche **1** bis **3**, wobei die biokompatible Matrix Kollagen umfasst.

5. Zusammensetzung nach einem der Ansprüche **1** bis **4**, wobei die biokompatible Matrix vom Menschen, Schwein, Rind oder Pferd ist.

6. Zusammensetzung nach einem der Ansprüche **1** bis 5, wobei mesenchymale Stammzellen von dem zu behandelnden Probanden stammen.

7. Zusammensetzung nach einem der Ansprüche **1** bis **6** zur Verwendung zur Behandlung einer Weichgewebeverletzung bei einem Probanden, der dieser bedarf.

8. Zusammensetzung zur Verwendung nach Anspruch **7**, wobei das Weichgewebe aus der Gruppe umfassend Hautgewebe, Muskelgewebe, dermales Gewebe, Sehnengewebe, Ligamentgewebe, Meniskusgewebe und Blasengewebe ausgewählt ist.

9. Zusammensetzung zur Verwendung nach Anspruch **7** oder **8**, wobei die Weichgewebeverletzung eine akute oder eine chronische Wunde ist.

10. Zusammensetzung zur Verwendung nach einem der Ansprüche **7** bis **9**, wobei die Weichgewebeverletzung aus der Gruppe umfassend Risse oder Brüche von Weichgewebe, Hautwunden, Hautverbrennungen, Hautgeschwüre, Operationswunden, Gefäßerkrankungen, Muskelerkrankung, Leistenbrüche und Strahlungswunden ausgewählt ist.

11. Zusammensetzung zur Verwendung nach Anspruch **10**, wobei die Hautwunde eine diabetische Wunde ist.

12. Zusammensetzung zur Verwendung nach einem der Ansprüche **7** bis **11**, wobei die Zusammensetzung topisch oder durch chirurgische Implantation verabreicht wird.

13. Verfahren zur Herstellung einer Zusammensetzung, umfassend eine biokompatible Matrix und eine Population von mesenchymalen Stammzellen, umfassend ein Inkubieren einer im Wesentlichen reinen Population von mesenchymalen Stammzellen mit einer biokompatiblen Matrix, wobei die mesenchymalen Stammzellen von Fettgewebe abgeleitet sind, wobei die mesenchymalen Stammzellen undifferenziert sind und wobei die Population von mesenchymalen Stammzellen weniger als 25 % Fibroblasten umfasst.

## Revendications

1. Composition comprenant une matrice biocompatible et une population de cellules souches mésenchymateuses, dans laquelle lesdites cellules souches mésenchymateuses sont dérivées de tissu adipeux, dans laquelle ladite population de cellules souches mésenchymateuses est substantiellement pure, dans laquelle lesdites cellules souches mésenchymateuses sont indifférenciées et dans laquelle ladite population de cellules souches mésenchymateuses comprend moins de 25% de fibroblastes.

2. La composition selon la revendication **1**, dans laquelle lesdites cellules souches mésenchymateuses sont dérivées de tissu adipeux sous-cutané.

3. La composition selon la revendication **1** ou **2**, dans laquelle ladite matrice biocompatible est acellulaire.

4. La composition selon l'une quelconque des revendications **1** à **3**, dans laquelle ladite matrice biocompatible comprend du collagène.

5. La composition selon l'une quelconque des revendications **1** à **4**, dans laquelle ladite matrice biocompatible est humaine, porcine, bovine ou équine.

6. La composition selon l'une quelconque des revendications **1** à **5**, dans laquelle lesdites cellules souches mésenchymateuses proviennent du sujet à traiter.

7. La composition selon l'une quelconque des revendications **1** à **6**, pour son utilisation pour le traitement d'une lésion de tissu mou chez un sujet qui en a besoin.

8. La composition pour son utilisation selon la revendication **7**, dans laquelle ledit tissu mou est sélectionné dans le groupe comprenant le tissu cutané, le tissu musculaire, le tissu dermique, le tissu tendineux, le tissu ligamentaire, le tissu le tissu méniscal and le tissu de la vessie.

9. La composition pour son utilisation selon la revendication **7** ou **8**, dans laquelle ladite lésion de tissu mou est une plaie aiguë ou chronique.

10. La composition pour son utilisation selon l'une quelconque des revendications **7** à **9**, dans laquelle ladite lésion de tissu mou est sélectionnée dans le groupe comprenant les fissures ou déchirures du tissu mou, les plaies cutanées, les brûlures cutanées, les ulcères cutanés, les plaies chirurgicales, les maladies vasculaires, les maladies musculaires, les hernies et les plaies de radiation.

11. La composition pour son utilisation selon la revendication **10**, dans laquelle ladite lésion cutanée est une plaie diabétique.

12. La composition pour son utilisation selon l'une quelconque des revendications **7** à **11**, dans laquelle ladite composition est administrée par voie topique ou par implantation chirurgicale.

13. Méthode de préparation d'une composition comprenant une matrice biocompatible et une population de cellules souches mésenchymateuses, comprenant l'incubation d'une population de cellules souches mésenchymateuses substantiellement pure avec une matrice biocompatible, dans laquelle lesdites cellules souches mésenchymateuses sont dérivées de tissu adipeux, dans laquelle lesdites cellules souches mésenchymateuses sont indifférenciées et dans laquelle ladite population de cellules souches mésenchymateuses comprend moins de 25% de fibroblastes.
